# EUROPEAN PATENT SPECIFICATION

(11) **EP 2 130 503 B1**
(45) Date of publication and mention of the grant of the patent: **13.04.2011**
(21) Application number: 09007322.2
(22) Date of filing: 02.06.2009
(51) Int. Cl.: A61B 17/128, A61B 17/122

(54) **Successive clipping device and manipulating handle**
Vorrichtung zur sukzessiven Platzierung von Klammern und Betätigungsgriff
Dispositif pour l'application successive de clips médicaux et manche de manipulation

(30) Priority: 03.06.2008 JP 2008145735; 06.08.2008 JP 2008203156; 10.09.2008 JP 2008232200; 29.09.2008 JP 2008251116
(43) Date of publication of application: 09.12.2009
(73) Proprietor: FUJIFILM Corporation, Minato-ku Tokyo (JP)
(72) Inventor: Iida, Takayuki, Ashigara-kami-gun Kanagawa (JP); Cui, Shengfu, Ashigara-kami-gun Kanagawa (JP)
(74) Representative: Klunker . Schmitt-Nilson . Hirsch

(56) References cited:
- WO-A-2006/030204
- US-A1- 2002 133 178
- US-A1- 2006 155 308

## Description

### BACKGROUND OF THE INVENTION

The present invention relates to an endoscopic clipping device used for stopping bleeding, closing a puncture, etc. in a living body or the like and, in particular, to a successive clipping device which allows a plurality of clips to be used in succession.

The present invention also relates to a manipulating handle for manipulating such a successive clipping device.

An endoscopic clipping device causes a clip to protrude from the forward end of an endocope inserted into a living body to pinch a bleeding portion or a portion to be treated after the removal of the lesion tissue with the clip, thereby stopping the bleeding or closing the puncture. In a conventionally used endoscopic clipping device, a single clip is detachably attached to the forward end of a manipulating wire, and each time clipping is performed, the entire sheath is pulled out the endoscope, and the sheath is loaded with the next clip before being inserted into the endoscope again for the next clipping. In this way, the conventional clipping device involves a rather bothersome operation.

In this connection, JP 2006-187391 A discloses an endoscopic clipping device allowing successive clipping.

Such an endoscopic clipping device includes a long sheath that accommodates a plurality of clips inside the forward end thereof. The endoscopic clipping device causes only a foremost clip among a plurality of clips to protrude from a forward end of the sheath, and causes the clip to perform a clipping manipulation (clipping) for stopping bleeding, closing a puncture, marking, etc. Then, the sheath is pulled to a rear end side by a predetermined length, whereby a next clip is placed in a state capable of performing clipping (is placed in a usable (standby) state) and the clipping can be performed successively.

The use of such an endoscopic clipping device enables clipping to be performed successively, which eliminates the necessity of performing a complicated work in which the entire sheath is pulled out the endoscope every time clipping is performed and a next clip is set and inserted again into the endoscope for the next clipping.

In an endoscopic clipping device disclosed in JP 2006-187391 A, in most cases, a clip is placed in a state capable of performing clipping by pulling a sheath to a rear end side by a predetermined length. However, such an endoscopic clipping device has the following problem: even if the sheath is pulled to a rear end side by a predetermined length in the same way, a clip may not be placed in a usable state, which makes it impossible to perform clipping.

For example, in such a clipping device, even if a foremost clip among a plurality of clips accommodated in a forward end portion of the sheath is placed at a position where the foremost clip is placed in a standby state capable of performing clipping at a time when the sheath is pulled to a rear end side by a predetermined length, before the foremost clip is inserted into a forceps mouth of the endoscope, the foremost clip is displaced from an original position in the sheath when the foremost clip is inserted into the forceps mouth which is inserted into a body cavity in a living body and curved; as a result, even if the sheath is pulled to the rear end side by a predetermined length, the clip is not placed in a usable state.

US 2006155308 corresponds to the preamble of claim 1, and discloses a clipping device which successfully metres out clips in a usable state.

### SUMMARY OF THE INVENTION

It is an object of the present invention to solve the above-mentioned problems in the related art and to provide a successive clipping device that can place a clip in a state capable of performing clipping stably at all the timers, as claimed in claim 1.

It is another object of the present invention to provide a manipulating handle for manipulating such a successive clipping device, as claimed in claim 7. Preferred embodiments are disclosed in the dependant claims.

A successive clipping device according to the present invention comprises: as claimed in claim 1, a plurality of clips loaded onto a forward end portion of a sheath while being engaged with another clips connected respectively in front and back directions; a plurality of connection rings corresponding to the plurality of clips and fitted into the sheath so as to be capable of advancing and retreating, and each covering an engagement portion between corresponding clips to maintain the plurality of clips in a connected state; a manipulating wire connected to a rearmost clip and pulling out a clip string formed of the plurality of clips; and a manipulating portion connected to a proximal end side of the sheath, which, regarding a foremost clip sinking in the sheath, moves the sheath and the manipulating wire relatively by a first movement amount required for allowing the foremost clip to protrude from a forward end of the sheath so as to place the foremost clip in a usable state, and which, regarding a subsequent clip, moves the sheath and the manipulating wire relatively by a second movement amount that is smaller than the first movement amount and required for allowing the subsequent clip to protrude from the forward end of the sheath so as to place the subsequent clip in a usable state.

In claims 1 and 7: A manipulating portion (claim 1) / handle (claim 7) for manipulating a successive clipping device loaded with a plurality of clips respectively connected in front and back directions according to the present invention comprises: a sheath; a handle main body fixed to a proximal end portion of the sheath; a manipulating wire having a forward end connected to the plurality of clips and a proximal end inserted in the sheath and the handle main body; a slider to which the proximal end of the manipulating wire is connected and which is placed movably in an extension direction of the sheath with respect to a manipulating portion main body; and a substantially cylindrical movement amount regulating member which is placed rotatably in a circumferential direction around an outer circumferential portion of the handle main body and which regulates a movement amount of the slider in the extension direction of the sheath in accordance with a rotation position in the circumferential direction.

### BRIEF DESCRIPTION OF THE DRAWINGS

FIGS. 1A and 1B are a partial side cross-sectional view and a partial front cross-sectional view, respectively, illustrating a successive clipping device according to Embodiment 1 of the present invention.
FIG. 2 is a perspective view illustrating a configuration of a clip used in Embodiment 1.
FIGS. 3A through 3C are a front view, a front cross-sectional view, and a bottom view of a connection ring used in Embodiment 1, respectively.
FIGS. 4A and 4B are a partial plan cross-sectional view and a partial front cross-sectional view, respectively, illustrating a schematic configuration of a manipulating portion used in Embodiment 1.
FIG. 5 is a partial enlarged view of FIG. 4B.
FIG. 6 is a partial enlarged view illustrating a manipulating portion according to a modified example.
FIG. 7 is a partial enlarged view illustrating a manipulating portion according to another modified example.
FIGS. 8A through 8E are partial cross-sectional views, respectively, illustrating the state of the successive clipping device in Embodiment 1 during clipping in stages.
FIGS. 9A and 9B are partial cross-sectional views, respectively, illustrating the predetermined state of the successive clipping device in Embodiment 1 during clipping.
FIGS. 10A and 10B are partial cross-sectional views, respectively, illustrating the use state of the successive clipping device in Embodiment 1.
FIG. 11 is a cross-sectional view illustrating a schematic configuration of a manipulating portion used in Embodiment 2.
FIG. 12 is a perspective view illustrating a main body rail used in the manipulating portion in FIG. 11.
FIGS. 13A and 13B are a perspective view of a slider guide used in the manipulating portion in FIG. 11 and a developed view of a proximal end of the slider guide, respectively.
FIG. 14 is a perspective view illustrating a position regulating member used in the manipulating portion in FIG. 11.
FIG. 15 is a developed view of the proximal end of the slider guide, schematically illustrating the positional relationship between the slider guide and a slider pin during clipping in Embodiment 2.
FIGS. 16A and 16B are a perspective view of a slider guide used in a manipulating portion in Embodiment 4 and a developed view of a proximal end of the slider guide, respectively.
FIGS. 17A through 17G are partial cross-sectional views, respectively, illustrating the state of a successive clipping device in Embodiment 4 during clipping in stages.
FIG. 18 is a developed view of a proximal end of a slider guide, schematically illustrating the positional relationship between the slider guide and a slider pin during clipping in Embodiment 4.
FIG. 19 is a partial front cross-sectional view illustrating a schematic configuration of a manipulating portion used in Embodiment 5.
FIG. 20 is a perspective view illustrating a manipulating portion in FIG. 19 with a slider guide removed.

### DETAILED DESCRIPTION OF THE PREFERRED EMBODIMENTS

In the following, preferred embodiments of the clipping device of the present invention are described with reference to the accompanying drawings.

### Embodiment 1

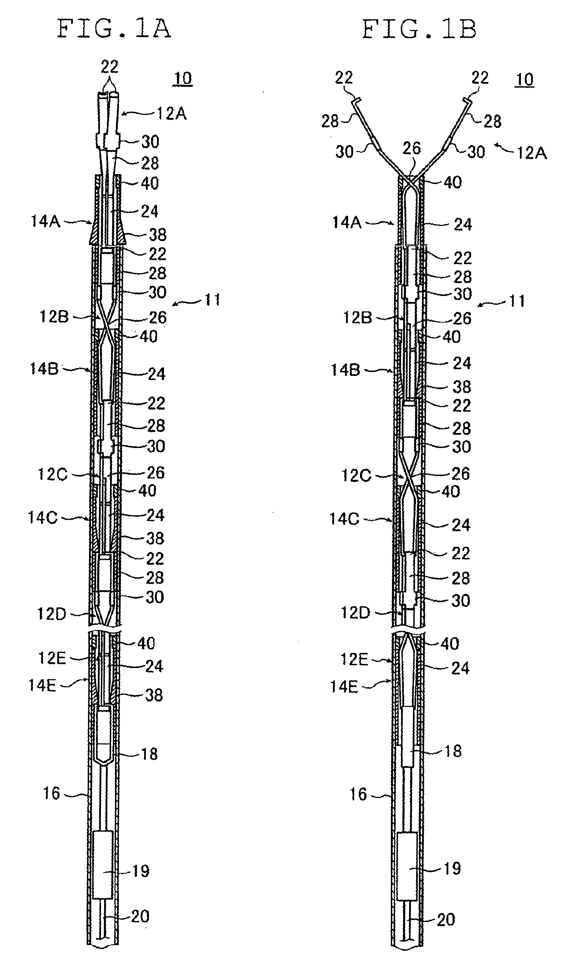

FIGS. 1A and 1B are sectional views of a clipping device 10 according to Embodiment 1 of the present invention, and FIG. 1B is a diagram as seen from an angle differing from FIG. 1A by 90 degrees. The clipping device 10 includes a manipulation operating portion 11. The manipulation operating portion 11 is composed of a plurality of clips 12 (12A, 12B, 12C, 12D, 12E), connection rings 14 (14A, 14B, 14C, 14D, 14E) that cover engagement portions of the adjacent clips 12 to maintain the connection state of the clips 12, a sheath 16 in which the clips 12 and the connection rings 14 are fitted, a dummy clip 18 connected to the trailing clip 12E, and a manipulating wire 20 connected to the dummy clip 18 via a connecting member 19.

FIGS. 1A and 1B illustrate an initial state (standby state) immediately before the start of clipping manipulation by the foremost clip 12.

One clip 12 and one connection ring 14 corresponding to the clip 12 form one endoscopic bleeding stop clip member, and the manipulation operating portion 11 includes a plurality of such bleeding stop clip members loaded into the interior of the distal end portion of the elongated sheath 16.

Further, the terminal end of the successive bleeding stop clip members is engaged with the dummy clip 18, and the dummy clip 18 is connected to the connecting member 19, through which the manipulating wire 20 and the plurality of clips 12 are connected to each other. Though not shown in FIGS. 1A and 1B, the manipulating wire 20 extends to the proximal end of the sheath 16 to be connected to a manipulating portion described later.

When the manipulating wire 20 is pulled out by a predetermined length by the manipulation of the manipulating portion, the series of clips 12 are moved by the same amount, and the foremost clip 12 is clamped by the connection ring 14 retaining the same, whereby clipping for stopping bleeding, marking, etc. is performed by the foremost clip 12. When the clipping by the foremost clip 12 has been completed, the sheath 16 is pulled to the manipulating portion side by a predetermined length, whereby the next clip 12 is placed in a usable state (standby state), thus making it possible to perform clipping successively.

While in FIGS. 1A and 1B the foremost clip 12A protrudes from the forward end of the sheath 16, when loading the plurality of clips 12 into the sheath 16, setting is made such that the foremost clip 12A is completely accommodated within the sheath 16. Further, while FIGS. 1A and 1B illustrate the 5-successive clipping device 10 in which 5 clips 12 are loaded in the sheath 16, the number of clips 12 may be any number not less than two.

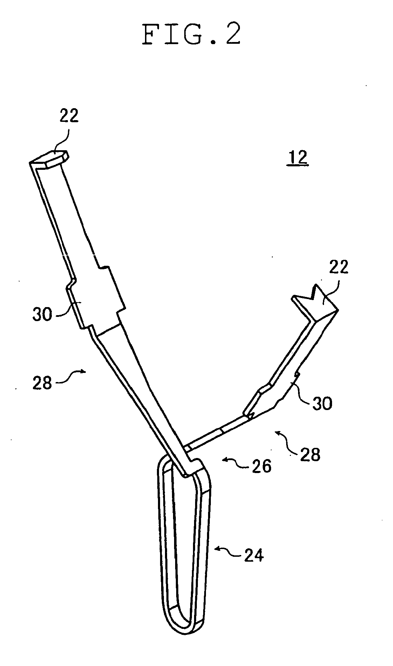

FIG. 2 is a perspective view of the clip 12. The clip 12 is a closed clip having a turned portion 24 turned by 180 degrees with respect to claw portions 22. That is, in forming the clip 12, a single elongated plate is bent by 180 degrees to form a closed end, and then both ends thereof are caused to cross each other at a crossing portion 26. Further, the end portions are bent so as to be opposed to each other, thereby forming the claw portions 22 to two open ends. On the open-end side of the crossing portion 26, there exist arm portions 28, 28, and, on the closed-end portion thereof, there exists the turned portion 24. At the central portion of each arm portion 28, 28, there are formed a partially widened projections 30, 30. The clip 12 may be formed of a metal with biocompatibility. For example, it is possible to use SUS 631, which is a spring stainless steel.

In the clip 12, the forward end portion (a clamping portion 40 described later) of the connection ring 14 fitted onto the crossing portion 26 moves by a predetermined amount toward the claw portions 22, 22 while pressurizing the arm portions 28, 28, whereby the arm portions 28, 28 and the claw portions 22, 22 are closed, with the claw portions 22, 22 exerting a predetermined fit-engagement force.

To reliably pinch an object of a bleeding portion or a portion to be treated after the removal of the lesion tissue, the claw portions 22, 22 are formed as V-shaped male type and female type ones. Further, as illustrated in FIG. 2, the arm portions 28 of the clips 12 gradually increase in width from the crossing portion 26 toward the projections 30.

The projections 30 have a width larger than that of the portions of the distal end side openings and the proximal end side openings of the connection ring 14 abutted by the projections 30. Thus, while the portions of the clip 12 other than the projections 30 can enter the interior of the connection ring 14, the projections 30 cannot enter the interior either from the distal end side or the proximal end side of the connection ring 14.

As illustrated in FIGS. 1A and 1B, the claw portions 22 of the second clip 12B are engaged with the turned portion 24 of the first clip 12A and retained by the connection ring 14A in a closed state, whereby the first clip 12A and the second clip 12B are connected together. As illustrated in FIG. 1A, the claw portions 22, 22 of the second clip 12B are engaged with the turned portion 24 of the first clip 12A in a direction orthogonal thereto, with the first clip 12A and the second clip 12B being differing in orientation by 90 degrees. Similarly, the clips 12C, 12D, and 12E are connected together, with their orientations alternately differing by 90 degrees.

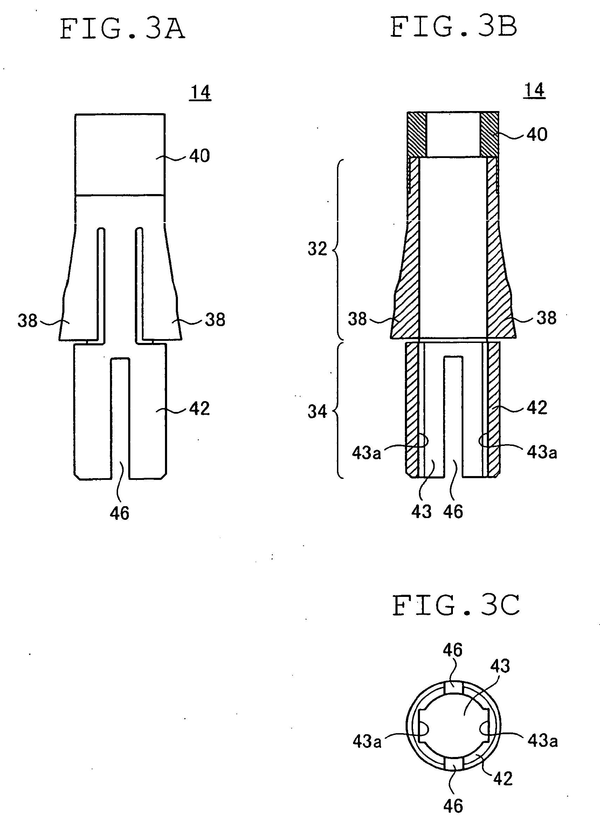

Each connection ring 14 is fitted into the sheath 16 so as to be capable of advancing and retreating while covering the engagement portion between two clips 12 and maintaining their connected state. That is, the outer diameter of the connection rings 14 is substantially the same as the inner diameter of the sheath 16 so that they can smoothly advance and retreat within the sheath 16 as the clips 12 move. FIGS. 3A through 3C illustrate the construction of each connection ring 14. FIG. 3A is a front view of the connection ring 14, FIG. 3B is a front cross-sectional view thereof, and FIG. 3C is a bottom view thereof.

The connection ring 14 includes a clamping portion 40 and a retaining portion 42. In the connection ring 14, the clamping portion 40 formed of metal is fixed to the forward end of the retaining portion 42 formed of resin, and the two members form an integral structure. The retaining portion 42 formed of resin serves to maintain the connected state and to retain the clip within the connection ring, and the clamping portion 40 formed of metal serves to clamp the clip. It is also possible for the connection ring 14 to be formed by a single member if it can provide the functions of both the clamping portion 40 and the retaining portion 42.

The clamping portion 40 is a cylindrical (ring-like) metal component mounted to the forward end side of the connection ring 14, and has a hole whose inner diameter is larger than the width of the portion of the clip 12 in the vicinity of the crossing portion 26 and smaller than the width of the projections 30. Thus, while the clamping portion 40 can move in the vicinity of the crossing portion 26 of the clip 12 it retains, it cannot be detached to the forward end side beyond the projections 30. That is, the projections 30 function as a stopper determining the movement limit of the connection ring 14 advancing with respect to the clip 12.

The clamping portion 40 is set at a predetermined position in the vicinity of the crossing portion 26 of the clip 12. The clamping portion 40 moves from its initial position, i.e., from the crossing portion 26 toward the projections 30, with the arm portions 28 of the clip 12 increasing in width, whereby it closes the arm portions 28, 28 of the diverging clip 12 to perform fixation and clamping. As the material of the clamping portion 40, a metal with biocompatibility, for example, a stainless steel SUS 304 is used. By forming the clamping portion 40 of metal, it is possible to exert on the metal clip 12 a frictional force, which serves as the clamping force.

The retaining portion 42 is a schematically cylindrical (ring-like) component formed by resin molding. The retaining portion 42 has a first region 32 retaining the preceding clip 12 and a second region 34 which is a connection retaining region retaining the next clip 12 while connected to the preceding clip.

The first region 32 has a large circular hole capable of accommodating the turned portion 24 of the clip 12 and larger than the hole of the clamping portion 40. On the outer surface of the forward end portion of the first region 32, there is formed a stepped portion onto which the clamping portion 40 is to be fitted, and the clamping portion 40 and the retaining portion 42 are fit-engaged with each other through close fit such that they are not detached from each other while loaded in the sheath 16 and during clipping manipulation. Further, the first region 32 has skirt portions 38 each diverging while inclined in a skirt-like fashion with respect to the axis of the connection ring 14 main body.

The forward end side, that is, the upper base portion of each skirt portion 38 as seen in FIGS. 3A and 3B is connected to the main body of the retaining portion 42, whereas the lower, diverging portion thereof is partially separated from the main body to be radially diverged or closed. Two skirt portions 38 are formed so as to be separated from each other by 180 degrees at the same position in the pulling direction for the clip 12, that is, in the vertical direction in FIGS. 3A and 3B.

When left as they are, that is, when in a state in which no external force is being imparted thereto, the skirt portions 38, 38 are diverged in a skirt-like fashion as illustrated in FIG. 3A. At this time, the interior of the first region 32 of the retaining portion 42 forms a columnar space as illustrated in FIG. 3B. When loading the connection rings 14 into the sheath 16, the following takes place: in the case, for example, of the second connection ring 14B illustrated in FIG. 1B, the skirt portions 38 are pushed in to enter the internal space, and the inner peripheral side portions of the skirt portions 38 pressurize the side surface (edge portion) of the turned portion 24 of the clip 12B retained by the first region 32, thus retaining the clip 12B such that it does not move in the rotating direction and the advancing/retreating direction within the connection ring 14B. It is also possible for the skirt portions 38 to pressurize and retain the clip retained by the second region 34, that is, the succeeding clip.

As in the case of the first connection ring 14A illustrated in FIG. 1A, the skirt portions 38, 38 extend beyond the forward end of the sheath 16, and are opened due to their own elasticity, releasing the retention of the clip 12A and becoming wider than the inner diameter of the sheath 16 to prevent the connection ring 14A from retreating into the sheath 16. In this state, the manipulating wire 20 is pulled, and the clip 12A retreats, whereby the connection ring 14A advances relative to the clip 12A to clamp the clip 12A.

Thus, it is necessary for the skirt portions 38 to have elasticity so that they can be closed inwardly within the sheath 16 and widen in a skirt-like fashion when they get out of the forward end of the sheath 16 and released from the external force. At the same time, it is also necessary for the skirt portions 38 to exhibit rigidity enabling them to retain the clip 12 within the sheath 16 and to withstand the repulsive force of the clamping force of the clip 12 at the forward end of the sheath 16.

From the above viewpoints, as the material of the retaining portion 42, there is used a material exhibiting biocompatibility and providing the requisite elasticity and rigidity for the skirt portions 38. As for their configuration, it is determined so as to satisfy the requirements in terms of elasticity and rigidity for the skirt portions 38. As the material of the retaining portion 42, it is possible to use, for example, polyphenylsulfone (PPSU). From the viewpoint of ease of production, it is desirable for the retaining portion 42 to be formed as an integral molding.

The second region 34 is provided on the proximal end side of the first region 32. The succeeding clip 12 engaged with the clip 12 retained by the first region 32 is retained in a state in which the claw portions 22, 22 thereof are closed while holding the closed end (tail portion) of the turned portion 24 of the preceding clip 12 therebetween.

The length of the second region 34 is substantially equal to the movement length required for the clamping portion 40 set at the initial position with respect to the clip 12 to move until the clamping of the clip 12 is completed. That is, while the clip 12 retreats relative to the connection ring 14 to be clamped, the second region 34 of the connection ring 14 maintains the connection between the two clips 12, 12 retained therein, allowing the pulling force of the rear clip 12 to be transmitted to the front clip 12, and when the clamping has been completed, the engagement portion of the two clips 12, 12 is detached from the second region 34, thereby canceling the connection between the clips 12, 12.

As illustrated in FIG. 3C, the second region 34 has a hole 43 having the same inner diameter as the proximal end side portion of the first region 32, and further, two grooves (recesses) 43a opposed to each other are formed. The grooves 43a, 43a can accommodate the arm portions 28, 28 of the clip 12 retained in the second region 34, with the claw portions 22, 22 being closed. Further, in the second region 34, slits 44 that are cut in from the proximal end are formed at two positions.

The grooves 43a, 43a are provided at two positions in the direction in which the claw portions 22 of the clip 12 retained in the second region 34 are opened and closed (horizontal direction in FIGS. 3B and 3C). The plate surfaces of the arm portions 28, 28 of the clip 12 retained in the second region 34 abut the inner walls of the grooves 43a, 43a. The width (opening width) of the grooves 43a is slightly larger than the maximum width of the arm portions 28 of the clip 12, and the distance from the wall surface of one groove 43a to the wall surface of the other groove 43a is substantially equal to the sum total of the lengths of the two claw portions 22, 22 of the clip 12 (length in the diverging direction). The width of the grooves 43a is smaller than the width of the projections 30 formed on the arm portions 28. Thus, the projections 30 of the clip 12 retained in the second region 34 cannot enter the grooves 43a.

The distance between the wall surfaces of the two grooves 43a is such that the engagement between the turned portion 24 of the preceding clip 12 and the claw portions 22, 22 of the next clip 12 is not canceled, and the distance is smaller than the sum total of the lengths of the two claw portions 22, 22 and the width of the portion of the turned portion 24 engaged with the claw portions 22, 22.

For example, the claw portions 22, 22 of the clip 12 retained in the second region 34 may slightly overlap each other, or the connection of the clip with the preceding clip 12 may be maintained, with a slight gap being left between the claw portions 22, 22.

The engagement portion between the two clips 12, 12 is retained in the portion of the second region 34 close to the boundary between the second region 34 and the first region 32. Inside the sheath 16, the turned portion 24 of the preceding clip 12 (e.g., the clip 12B in the connection ring 14B illustrated in FIG. 1B) is retained by the closed skirt portions 38 in the first region 32, and hence the advancing/retreating movement and rotating movement of the clip is restrained. The next clip 12 (e.g., the clip 12C in the connection ring 14B illustrated in FIG. 1B) engaged with the preceding clip 12 is retained in an orientation differing by 90 degrees from the preceding clip by the grooves 43a of the second region 34, whereby rotating movement of the clip is restrained, and the clip is engaged with the preceding clip restrained in advancing/retreating movement, thereby restraining the advancing/retreating movement thereof. That is, the engagement portion between the front and rear clips is retained by the connection ring 14 with very little play.

The slits 46 are formed at two positions deviated from the skirt portions 38, 38 by 90 degrees so as to be shallower than the upper end of the second region 34. In other words, the slits 46 are provided at positions deviated by 90 degrees from the direction in which the clips 12 retained by the second region 34 are diverged.

Due to the provision of the slits 46, the connection ring 14 is improved in terms of flexibility, and the clipping device 10 can pass a curved portion of small curvature. Further, due to the provision of the slits 46, the hem (proximal end portion) of the connection ring 14 is partially turned up, and hence, when the front and rear clips 12 are connected together prior to the loading of the clips 12 into the sheath 16, the connection is advantageously facilitated through the turning of the hem of the connection ring 14.

The slits 46 are situated so as to be shallower than the skirt portions 38, whereby a substantial reduction in the strength of the connection ring 14 is prevented. Further, the depth of the slits 46 is shallower than the position of the rear end of the clip 12 retained in the first region 32, that is, shallower than the engagement position of the clips 12, 12, and hence, also in the connection clip unit prior to the loading into the sheath 16, it is possible to maintain the retention of the clip 12 in the second region 34 of the connection ring 14.

As illustrated in FIGS. 1A and 1B, the claw portions 22 of the second clip 12B are engaged with the turned portion 24 of the first clip 12A, and the engagement portion is retained by the connection ring 14A. The claw portions 22, 22 of the second clip 12B are retained in the closed state by the inner wall of the connection ring 14A (second region 34 thereof). As a result, the connection of the first clip 12A and the second clip 12B is maintained. Similarly, the connection of the second clip 12B and the third clip 12C is maintained by the connection ring 14B, the connection of the third clip 12C and the fourth clip 12D is maintained by the connection ring 14C, the connection of the fourth clip 12D and the fifth clip 12E is maintained by the connection ring 14D, and the connection of the fifth clip 12E and the dummy clip 18 is maintained by the connection ring 14E.

The rearmost clip 12E is engaged with the dummy clip 18, which is not used for clipping. At its forward end portion, the dummy clip 18 has a resilient portion of a configuration similar to that of the open end side half as from the crossing portion of the clip 12. The resilient portion is engaged with the turned portion of the clip 12E, with the claw portions thereof being closed, and releases the clip 12E when the claw portions are opened. At the proximal end portion of the dummy clip 18, there exits the connecting member 19, to which the manipulating wire 20 is connected.

The sheath 16 is formed, for example, of a flexible coil sheath formed through intimate winding of metal wire. The sheath 16 allows the clip 12 to be movably fitted therein on the forward end side and accommodates the manipulating wire 20 connected to the clip 12 via the dummy clip 18 and the connecting member 19, and is connected to the manipulating portion on the proximal end side. The inner diameter of the sheath 16 is one allowing canceling of the engagement between the turned portion 24 of the preceding clip 12 and the claw portions 22, 22 of the next clip 12. That is, the inner diameter of the sheath 16 is larger than the sum total of the lengths of the two claw portions 22, 22 and the width of the portion of the turned portion 24 engaged with the claw portions 22, 22.

The manipulating wire 20 allows the plurality of clips 12 to advance or retreat in a series of clipping manipulations, and for example, is formed of a metal wire. The manipulating wire 20 is accommodated in the sheath 16. One end of the manipulating wire 20 is connected to the clip 12 via the connecting member 19 and the dummy clip 18, and the other end thereof extends to the proximal end of the sheath 16 to be connected to the manipulating portion. Further, the proximal end of the sheath 16 is also attached to the manipulating portion together with the manipulating wire 20.

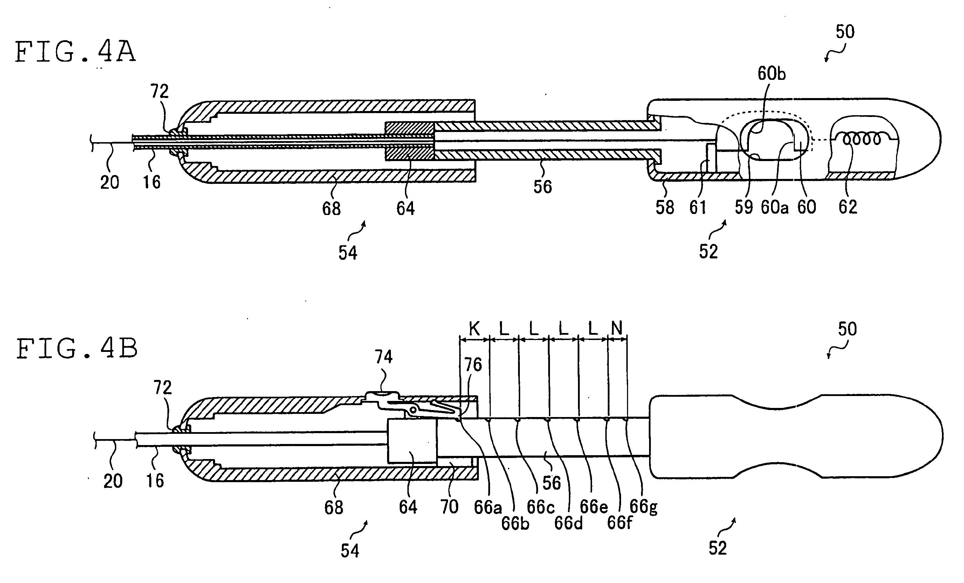

FIGS. 4A and 4B are a partial plan cross-sectional view and a partial front cross-sectional view, respectively, illustrating the configuration of the manipulating portion 50 used in Embodiment 1. In FIGS. 4A and 4B, the left side corresponds to the forward end side connected to the clipping device 10, and the right side corresponds to the rear end side (or the proximal end side) to be manipulated by an operator.

A manipulating portion 50 includes a wire manipulating handle 52 constituting a manipulating portion main body, and a sheath manipulating handle 54 serving as a grasping portion for grasping the proximal end portion of the sheath 16, with the sheath manipulating handle 54 being slidable with respect to the wire manipulating handle 52.

The wire manipulating handle 52 includes a cylindrical case 58, a positioning pipe 56 fixed coaxially to the forward end of the case 58, and a lever 60 and a spring 62 retained inside the case 58.

The lever 60 is retained inside the case 58 so as to be movable in the longitudinal direction (axial direction of the wire manipulating handle 52). Parts of a forward end side and a rear end side of the lever 60 appear through a window 59 provided at the center portion of the case 58. The operator can place his/her finger onto a part of the lever 60 on the forward end side to push the lever 60 to the forward end side and can hook his/her finger onto a part of the lever 60 on the rear end side to pull the lever 60 to the rear end side. The spring 62 is attached to the rear end of the lever 60.

The spring 62 is compressed by pulling the lever 60 rearwards, and when the pulling force on the lever 60 is released, the spring 62 forwardly pushes back the lever 60 by repulsive force. As a result, the lever 60 is restored to the former position (home position).

Further, the spring 62 is pulled when the lever 60 is pushed forwards, and when the pushing force on the lever 60 is released, the spring 62 rearwardly pushes back the lever 60 by repulsive force. As a result, the lever 60 is restored to the former position (home position).

The forward and rearward movement limit for the lever 60 is determined by the window 59. That is, the position where a surface 60b of the lever 60 onto which the finger is placed coincides with the forward end of the window 59 is the forward movement limit for the lever 60, and the position where a surface 60a of the lever 60 onto which the finger is hooked coincides with the rear end of the window 59 is the rearward movement limit for the lever 60. It is also possible to provide a regulating plate at the rear of the lever 60, and to determine the rearward movement limit for the lever 60 through abutment of the rear end of the lever 60 against the regulating plate.

A regulating plate 61 is provided in front of the lever 60 to determine the home position of the lever 60. Therefore, when the lever 60 is pulled to the rear end side, the lever 60 is urged by the spring 62 and moves forwards until it abuts the regulating plate 61 to return to the home position. Further, in this embodiment, the regulating plate 61 is formed of a member that is not at least urged by the spring 62 to move, but is curved forwards with a force generated when the lever 60 is pushed forwards. Therefore, when the lever 60 is pushed to the forward end side, the lever 60 causes the regulating plate 61 to be curved and moves the forward end of the regulating plate 61 forwards, and thereafter, the regulating plate 61 is restored to the former position due to the elasticity, whereby the lever returns to the home position.

In this way, the lever 60 can move longitudinally by a fixed amount between the home position and the rearward movement limit.

While in FIG. 4A the spring 62 is formed of a coil spring, this should not be construed restrictively. It is only necessary for the spring 62 to be capable of forwardly urging the lever 60. Thus, it is also possible to use a plate spring or some other elastic member as urging means.

Fixed to the forward end of the lever 60 is the manipulating wire 20 for pulling the clips 12. The manipulating wire 20 extends through the sheath manipulating handle 54 and the positioning pipe 56 to reach the lever 60.

When the operator inserts his/her finger into the window 59 and pulls the lever 60 to move the lever 60 rearwards, the manipulating wire 20 attached to the forward end of the lever 60 also moves, and the forward end of the manipulating wire 20 moves rearwards. When the pulling force applied to the lever 60 is canceled and the lever 60 is restored to the former position, the manipulating wire 20 also moves, with its forward end returning to the former position.

On the other hand, when the operator inserts his/her finger into the window 59 and pushes the lever 60 to move the lever 60 forwards, the manipulating wire'20 attached to the forward end of the lever 60 also moves, and the forward end of the manipulating wire 20 moves forwards. When the pushing force applied to the lever 60 is canceled and the regulating plate 61 is restored to the former position, the manipulating wire 20 also moves, with its forward end returning to the former position.

The pulling amount of the manipulating wire 20 in the clipping is a very small amount, e.g., 3.1 mm to 3.2 mm. Thus, in order to give a reliable operational feel at the manipulating portion 50, a pulling amount magnifying mechanism for the manipulating wire 20 may be provided between the pulling amount of the manipulating wire 20 and the manipulating amount of the lever 60, making the movement amount of the lever 60 a predetermined number of times of the movement amount of the manipulating wire 20.

The positioning pipe 56 is a hollow pipe-like member constituting a slide mechanism which allows a sheath manipulating handle 54 to move forwards and rearwards with respect to the wire manipulating handle 52, and through which the manipulating wire 20 passes. The inner diameter of the positioning pipe 56 is larger than the outer diameter of the sheath 16, making it possible to insert the sheath 16 into the positioning pipe 56.

Here, as illustrated in FIG. 4B, a plurality of (7 in the illustrated example) notches 66 (66a through 66g) used for pulling the sheath 16 by a predetermined length are formed in the upper surface of the positioning pipe 56.

The notches 66 (66a through 66g) are formed in the axial direction from the forward end side to the rear end side of the positioning pipe 56 so that the interval between the first notch 66a and the second notch 66b is a predetermined interval K, each interval between the adjacent notches 66 from the second notch 66b to the sixth notch 66f is a predetermined interval L, and the interval between the sixth notch 66f and the seventh notch 66g is a predetermined interval N.

The predetermined intervals K, L, and N are described later.

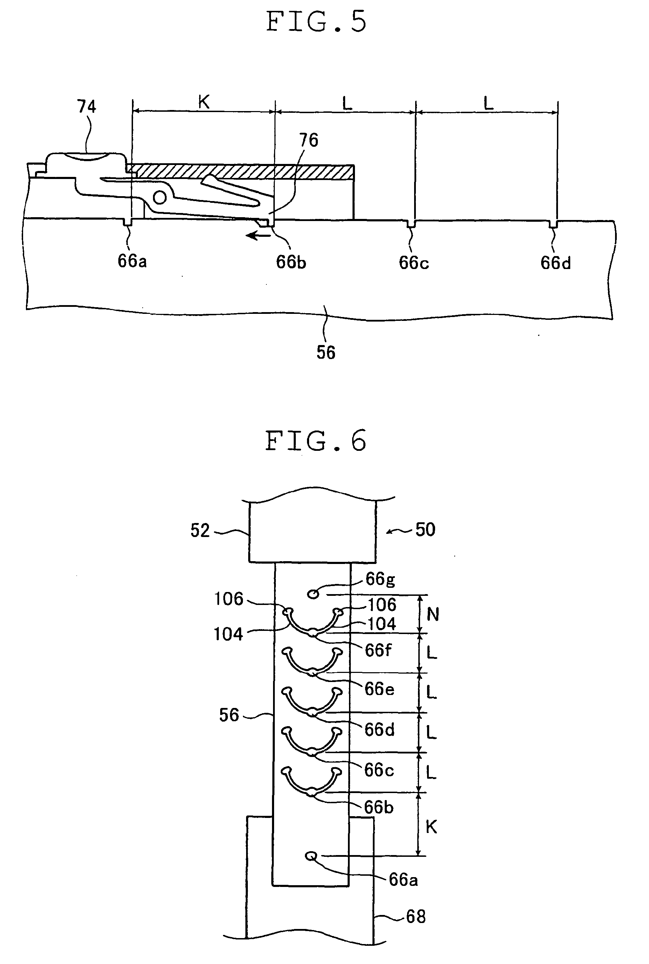

As illustrated in FIG. 5, the second notch 66b has a predetermined length in the axial direction of the positioning pipe 56 so that a claw 76 described later can move forwards and rearwards in the notch 66b.

Further, as illustrated in FIG. 6, in the upper surface of the positioning pipe 56, grooves 104 in which the claw 76 is moved are formed in an arc shape in the right and left directions of each notch 66 from the second notch 66b to the sixth notch 66f among the notches described above so that the sheath 16 is rotated in the left or right direction, and a notch 106 is formed at each forward end of the grooves 104. The distance in the axial direction of the positioning pipe 56 between the notch 66 and the notch 106 is, for example, about 1 mm.

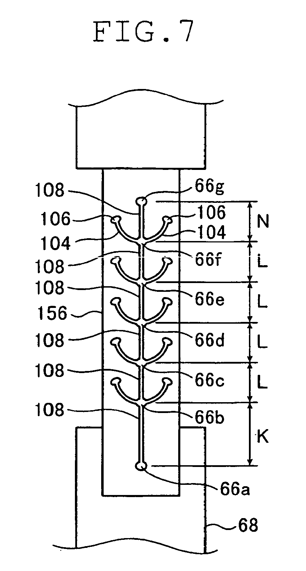

As in a positioning pipe 56 illustrated in FIG. 7, grooves 108 allowing the claw 76 to slide may be provided between the adjacent notches 66.

In this case, the diameter of the notch 106 is formed to be larger than the width of the groove 104 so that the claw 76 slides on the groove 104 to be caught by the notch 106.

As illustrated in FIG. 4A, the forward end portion of the positioning pipe 56 is inserted into the sheath manipulating handle 54, and a detachment prevention ring 64 is attached to the forward end portion thereof. In the center portion of the detachment prevention ring 64, a hole slightly larger than the outer diameter of the sheath 16 is formed. The detachment prevention ring 64 retains the sheath 16 movably in the axial direction.

As illustrated in FIGS. 4A and 4B, the sheath manipulating handle 54 has a cylindrical case 68, a support block 70, and a sheath retaining ring 72.

The support block 70 is arranged at the rear end of the sheath manipulating handle 54, and slidably supports the positioning pipe 56 inserted into the sheath manipulating handle 54. Further, as illustrated in FIG. 4B, the forward end side surface of the support block 70 abuts the detachment prevention ring 64 attached to the forward end of the positioning pipe 56, preventing the positioning pipe 56 from being detached from the sheath manipulating handle 54.

The sheath retaining ring 72 is provided at the forward end of the case 68 on the axis of the sheath manipulating handle 54, and fixedly retains the outer periphery of the sheath 16 inserted into the sheath manipulating handle 54. Thus, when the sheath manipulating handle 54 moves, the sheath 16 also moves together with the sheath manipulating handle 54.

The sheath manipulating handle 54 further includes a button 74 protruding out of the case 68 and a claw 76 provided inside the case 68 and interlocked with the movement of the button 74, which constitute a slide mechanism for allowing the sheath manipulating handle 54 to slide forwards and rearwards with respect to the wire manipulating handle 52. The claw 76 is urged so as to be pressed against the positioning pipe 56, and is caught by the notches 66 of the positioning pipe 56, thereby performing positioning on the sheath manipulating handle 54 with respect to the wire manipulating handle 52 and stopping the movement thereof.

When the button 74 is depressed, the claw 76 is raised and detached from the notches 66, enabling the wire manipulating handle 52 to move with respect to the sheath manipulating handle 54. When the hand is released from the button 74 and the sheath manipulating handle 54 is moved with respect to the wire manipulating handle 52, its movement is stopped when the claw 76 is caught by the next notch 66. Thus, assuming that the interval K, L, or N between the adjacent notches 66 is one stroke, the sheath manipulating handle 54 and the sheath 16 can move by the stroke length K, L, or N.

Further, in Embodiment 1, as illustrated in FIG. 6, the claw 76 is moved along the grooves 104 from the second to sixth notches 66b to 66f, whereby the sheath manipulating handle 54 and the sheath 16 can be rotated in the left or right direction.

When the sheath 16 moves in the axial direction with the movement of the sheath manipulating handle 54, the proximal end portion of the sheath 16 advances through the hole of the detachment prevention ring 64 to enter the interior of the positioning pipe 56.

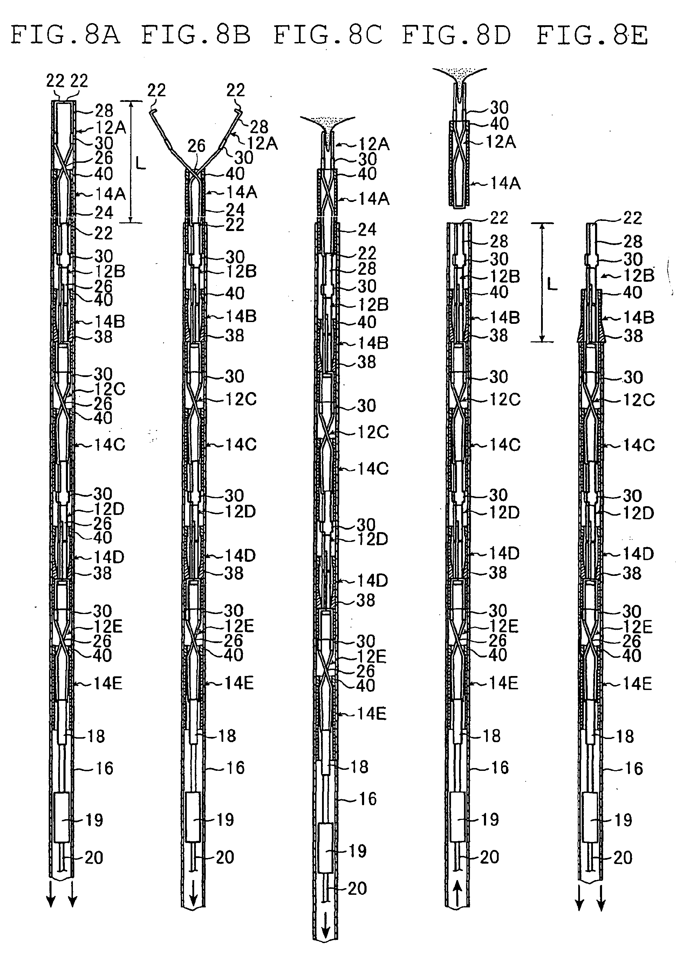

Next, the operation of the clipping device 10 during clipping is described with reference to FIGS. 8A through 8E.

First, as illustrated in FIG. 8A, after five bleeding stop clip units (hereinafter simply referred to as clipping units) formed of the clips 12A through 12E and the connection rings 14A through 14E have been loaded into the sheath 16, the sheath 16 is inserted into the forceps channel of an endoscope. As illustrated in FIG. 8A, the forward end of the clip 12A is substantially matched with the forward end of the sheath 16.

The foremost clip 12A is retained in the closed state by the inner wall of the sheath 16. Each of the connection rings 14A through 14E is fitted such that the clamping portion 40 thereof is situated at the initial position, i.e., in the vicinity of the crossing portion 26 of each of the clips 12A through 12E. At this time, the upper ends of the projections 30 of the clips 12B through 12E are respectively situated directly below the connection rings 14A through 14D.

When the forward end of the sheath 16 reaches the forward end of the insert portion of the endoscope inserted into the living body, and protrudes from the forward end of the endoscope, in the manipulating portion 50 illustrated in FIG. 4B, the sheath manipulating handle 54 is pulled such that the claw 76 of the sheath manipulating handle 54 moves by the predetermined interval K from the first notch 66a to the second notch 66b. Because the sheath 16 is fixed to the sheath manipulating handle 54, the sheath 16 retreats by the same amount as the movement amount L of the sheath manipulating handle 54 (i.e., predetermined interval K). Through this manipulation, solely the sheath 16 is pulled to the manipulating portion side, with the manipulating wire 20 remaining stationary.

Hereinafter, an interval K from the first notch 66a to the second notch 66b, i.e., a predetermined pulling length K is described.

As illustrated in FIG. 8A, in the case where the forward end of the clip 12A is substantially matched with the forward end of the sheath 16 when the sheath 16 is inserted into a forceps channel of the endoscope, all the intervals between the adjacent notches 66 are set to be a load interval L of the clips 12, whereby the sheath 16 can be moved to the manipulating portion 50 side by the distance L and the plurality of clips 12 can be placed in a usable state successively by moving the claw 76 by one stroke from one notch 66 to the adjacent notch 66.

Thus, in terms of the design, when the sheath 16 with the clip members loaded therein is inserted into the forceps channel of the endoscope, and after the sheath 16 reaches the forward end portion of the endoscope, the claw 76 is moved from the first notch 66a to the second notch 66b and the sheath 16 is pulled to the manipulating portion 50 side by one stroke, i.e., the load interval L of the clips 12, whereby the foremost clip 12A should be placed in a usable state. However, as described above, in the case where the foremost clip is displaced from the original position in the sheath due to the curved shape and the like of the forceps mouth of the endoscope into which the sheath 16 is inserted, the clips 12 may not be placed in a usable state, which makes it impossible to perform clipping.

The inventors of the present invention have earnestly studied and found the following case.

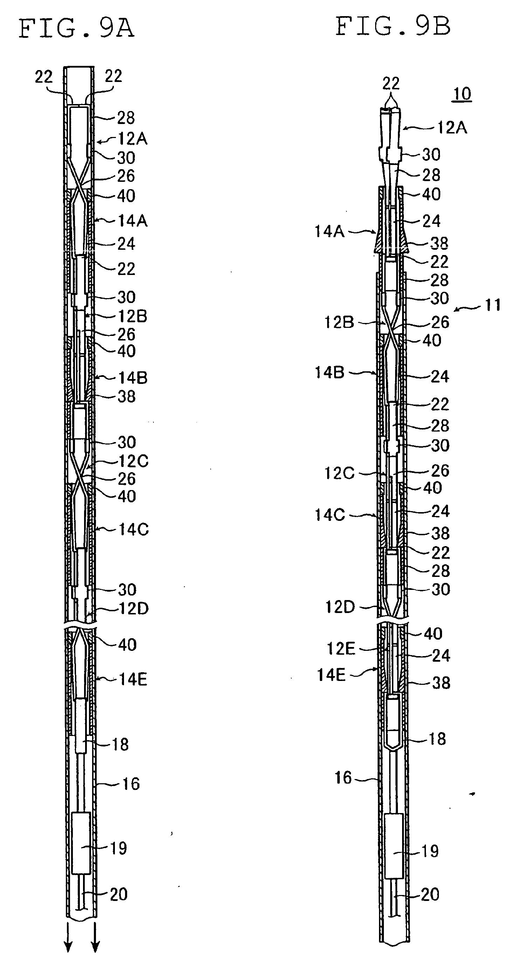

Usually, the distance between the proximal end side (manipulating portion side) of the sheath 16 and the forward end side thereof is several meters (for example, about 2 meters). Therefore, when the sheath 16 is curved, the manipulating wire 20 passes through a portion outside of the curve instead of the center axis portion in the sheath 16 in the curved portion. As a result, the axial positions of the sheath 16 and the manipulating wire 20 are displaced, and as illustrated in FIG. 9A, the foremost clip 12A may sink inside the sheath 16.

Then, the inventors of the present invention found the following. In the above-mentioned case, even if the sheath 16 is moved to the manipulating portion 50 side by the load interval L of the clips 12, the skirt portions 38 of the clip 12A do not protrude completely from the sheath 16, i.e., the clip 12A is not placed in a state capable of performing clipping.

The distance between the forward end of the clip 12A having sunk in the sheath 16 and the forward end of the sheath 16 (hereinafter, which may be referred simply as sinking amount) is, for example, at most about 3.5 mm, though varying depending upon the curved state of the sheath 16.

In Embodiment 1, assuming that the foremost clip 12A sinks in the sheath 16, the interval K from the first notch 66a to the second notch 66b is set to be a length obtained by adding the previously calculated sinking amount of the clip 12A to the load interval L of the clips 12.

Thus, even in the case where the foremost clip 12A sinks in the sheath 16 when the sheath 16 with the clip members loaded therein is inserted into the forceps channel of the endoscope and the sheath 16 reaches the forward end portion of the endoscope, the claw 76 is moved from the first notch 66a to the second notch 66b and the sheath 16 is moved to the manipulating portion 50 side by the interval K of those notches, whereby the foremost clip 12A accommodated in the sheath 16 can be placed in a state capable of performing clipping.

In this manner, the distance from the first notch 66a to the second notch 66b is set to be a predetermined interval (predetermined pulling length) K as described above so as to cancel the sinking amount of the foremost clip 12A in the sheath 16, whereby the second and subsequent clips 12 do not sink in the sheath 16 when the clipping of the foremost clip 12A is completed.

For example, when the load interval L of the clips 12 is 12.4 mm, and the previously calculated sinking amount of the clip 12A is 3.1 mm, the predetermined interval (predetermined pulling length) K is 15.5 mm.

In the case where the actual sinking amount of the clip 12A is smaller than the previously calculated sinking amount for the reason that the sheath 16 is not so covered as expected, etc., when the claw 76 is moved from the first notch 66a to the second notch 66b to move the sheath 16 to the manipulating portion side by the predetermined pulling length K, as illustrated in FIG. 9B, a gap is formed between the lower ends of the skirt portions 38 of the connection ring 14A corresponding to the clip 12A and the forward end of the sheath 16.

In such a case, it is preferred that the sheath 16 is pushed out to the side opposite to the manipulating portion 50 to bury the gap between the lower ends of the skirt portions 38 and the forward end of the sheath 16.

For example, as illustrated in FIG. 5, the second notch 66b from the forward end side has a predetermined length in the axial direction of the wire manipulating handle 52 so that the claw 76 moves forwards and rearwards in the second notch 66b. Therefore, the claw 76 is pushed back in the direction opposite to the manipulating portion 50 (in the direction indicated by an arrow of FIG.5) in the second notch 66b to push out the sheath 16 to the forward end side, whereby the gap between the lower ends of the skirt portions 38 of the connection ring 14A and the forward end of the sheath 16 can be buried.

Alternatively, the gap formed between the lower ends of the skirt portions 38 of the connection ring 14A and the forward end of the sheath 16 may be buried by pulling the manipulating wire 20 to the manipulating portion 50 side with respect to the sheath 16.

Further, even in the case where the skirt portions 38 of the foremost connection ring 14A are not opened due to the curved state or the like of the sheath 16 even by setting the distance from the first notch 66a to the second notch 66b to be the above-mentioned predetermined interval (predetermined distance) K, it is preferred to push out the manipulating wire 20 slightly to the forward end side.

For example, in FIG. 4A, the manipulating wire 20 can be pushed out to the forward end side by placing the finger on the surface 60b of the lever 60 exposed from the window 59 to push out the lever 60 to the forward end side.

Alternatively, in the case where the skirt portions 38 of the foremost connection ring 14A are not opened, the sheath 16 may be slightly moved to the manipulating portion 50 side, for example, by about 1 mm by rotating the sheath 16 to the right or left.

For example, in FIG. 6, the claw 76 is moved to the notch 106 along the groove 104 in the right or left direction from the notch 66b, whereby the sheath 16 can be rotated to the right or left to move the sheath 16 to the manipulating portion 50 side.

As described above, when the skirt portions 38 of the foremost connection ring 14A protrude from the forward end of the sheath 16, the skirt portions 38 of the connection ring 14A are opened outwards, and the claw portions 22, 22 of the clip 12A protruding from the sheath 16 spread due to the urging force to be placed in a state illustrated in FIG. 8B. This places the foremost clip 12A in a usable state. In FIG. 8B, because the skirt portions 38 of the connection ring 14A are placed in a direction perpendicular to the pulling surface, the skirt portions 38 do not appear in the figure.

The connecting portion between the clip 12A and the clip 12B is situated directly below the skirt portions 38 of the connection ring 14A, and hence, in the state as illustrated in FIG. 8B, the forward end of the clip 12B is substantially matched with the forward end of the sheath 16.

When the sheath 16 is pulled, there is exerted a frictional force between the sheath 16 and the connection rings 14A through 14E fitted into the sheath 16. However, between the connection rings 14A through 14E and the clips 12A through 12E, there are exerted the pressurizing force of the clips 12 due to the inner side portions of the closed skirt portions 38, and the pressurizing force applied to the inner wall surfaces of the connection rings 14 (second regions 34 thereof, see FIG. 3B) due to the resilient force of the claw portions 22 of the succeeding clips 12 inclined to open. Further, the projections 30 of the clips 12B through 12E abut the proximal ends of the connection rings 14A through 14D, and cannot enter the holes 43 of the connection rings 14 (see FIG. 3B). Thus, even if the sheath 16 is pulled, the connection rings 14A through 14E make no unnecessary movement. Thus, the connection rings 14A through 14E can maintain the state in which they respectively retain the clips 12A through 12E.

Next, the clipping device 10 in the state of FIG. 8B is moved to press the claw portions 22, 22 of the diverged clip 12A against the portion to be subjected to clipping, and the lever 60 of the manipulating portion 50 (see FIGS. 4A and 4B) is pulled, whereby the manipulating wire 20 is pulled by a predetermined amount. By pulling the manipulating wire 20, the clips 12A through 12E engaged sequentially starting from the dummy clip 18 are pulled all together.

At this time, in the states illustrated in FIGS. 8B and 8C, the skirt portions 38 of the connection ring 14A protruding from the forward end of the sheath 16 are opened, and the retention of the clip 12A by the pressure of the skirt portions 38 is cancelled. Further, because the skirt portions 38 are opened at the forward end of the sheath 16, the connection ring 14A is prevented from retreating into the sheath 16. Therefore, as illustrated in FIG. 8C, the foremost clip 12A retreats with respect to the connection ring 14A, when the manipulating wire 20 is pulled. The lower ends of the projections 30 of the clip 12A are pushed in the forward end of the connection ring 14A, i.e., the clamping portion 40, whereby clamping of the clip 12A by the connection ring 14A is completed.

As illustrated in FIG. 9B, in the case where a gap is formed between the lower ends of the skirt portions of the clip 12A and the forward end of the sheath 16, the lower ends of the projections 30 of the clip 12A can be pressurized against the forward end of the connection ring 14A and the lower ends of the skirt portions 38 of the connection ring 14A can be pressurized against the forward end of the sheath 16 by pulling the lever 60 to the rear end side with a force larger than that for performing ordinary clipping.

At this time, the inner diameter of the sheath 16 is larger than the outer diameter of the second region 34 of the retaining portion 42 of each of the connection rings 14, and there is a slight gap between the inner side surface of the sheath 16 and the second region 34 of the retaining portion 42 of each of the connection rings 14. Therefore, the gap between the lower ends of the skirt portions 38 of the connection ring 14A and the forward end of the sheath 16 can be buried relatively easily without causing a friction between the sheath 16 and the connection rings 14 after the clamping of the clip 12A by the connection ring 14A is completed by pulling the lever 60 to the rear end side with a force larger than that for performing ordinary clipping.

The engaged portion between the clip 12A and the next clip 12B comes off from the rear end of the connection ring 14A at the same time as the clamping of the clip 12A by the connection ring 14A. When the engaged portion between the clip 12A and the clip 12B comes off from the connection ring 14A, the arm portions 28 of the clip 12B are diverged until they abut the inner wall of the sheath 16 due to a resilient force, and the claw portions 22, 22 of the clip 12B are diverged more widely than the width of the turned portion 24 of the clip 12A, whereby the connection between the clip 12A and the clip 12B is cancelled. This enables the clip 12A and the connection ring 14A to be detached from the sheath 16, whereby the clipping by the clip 12A and the connection ring 14A is completed.

On the other hand, the succeeding clips 12B through 12E are retained by the connection rings 14B through 14E whose skirt portions 38 are closed so as not to move in the rotating direction and the advancing/retreating direction with respect to the connection rings 14B through 14E. Further, the claw portions 22 are pressed against the inner walls of the second regions 34 (see FIG. 3B) of the connection rings 14B through 14E by the expanding force (urging force) of the claw portions 22 of the clips 12C through 12E engaged with the clips 12B through 12E and the claw portions of the dummy clip 18, with the result that the frictional force between the clips 12B through 12E and the connection rings 14B through 14E is enhanced. Thus, the connection rings 14B through 14E move with the movement of the clips 12B trough 12E.

That is, the clips and the connection rings other than the foremost clip 12A and the connection ring 14A retaining the same, i.e., the clips 12B through 12E and the connection rings 14B through 14E advance and retreat integrally with respect to the sheath 16, and the connected state of the clips 14B through 14E and the dummy clip 18 is maintained by the connection rings 14B through 14E.

The manipulating wire 20 is constructed so as to be capable of being pulled by a fixed amount from the initial state. This fixed amount is an amount equal to the length of the second regions 34 of the connection rings 14 or an amount slightly larger than that, and at the same time, it is an amount equal to the length from the lower ends of the projections 30 of each clip 12 to the forward end of the connection ring 14 retaining that clip 12, or an amount slightly smaller than that. In the manipulating portion 50 of FIG. 4A, this fixed amount is determined by the length as measured from the home position of the lever 60 to the movement limit at the rear.

After it has been pulled by the fixed amount, the manipulating wire 20 is soon restored by that fixed amount due to the spring 62 urging the lever 60 of the manipulating portion 50. When the pulling force of the lever 60 is canceled at the manipulating portion 50, the lever 60 is restored to the former position, and the manipulating wire 20 pulled from the state illustrated in FIG. 8B to the state illustrated in FIG. 8C is thereby restored to the former position, whereby the state as illustrated in FIG. 8D is attained. That is, as in the case of FIG. 8B, the forward end of the second clip 12B is restored to the position where it is substantially matched with the forward end of the sheath 16.

Next, by moving the claw 76 from the second notch 66b to the third notch 66c in the manipulating portion 50 in FIG. 4A in order to place the second clip 12B in a usable state, the sheath 16 is pulled to the manipulating portion side by predetermined one stroke, i.e., by a predetermined interval (predetermined pulling length) L.

The predetermined interval L refers to the distance between the forward ends of adjacent two clips 12 loaded in the sheath 16, i.e., the load interval of the clips 12 in the sheath 16, as illustrated in FIG. 8D.

The predetermined interval L corresponds to the length from the second notch 66b to the third notch 66c, i.e., the length of one stroke by which the sheath 16 is pulled. Therefore, when the claw 76 is moved from the second notch 66b to the third notch 66c, the sheath 16 moves to the manipulating portion side by the predetermined interval L, and the clip 12B shifts from the state in which the position of the forward end is substantially matched with the position of the forward end of the sheath 16 as illustrated in FIG. 8D to the state in which the clipping can be performed as illustrated in FIG. 8E.

After that, the manipulating wire 20 is pulled by a predetermined amount with the claw portions of the clip 12B being pressed against a site desired to be subjected to clipping in the same way as in the clip 12A described above. This cancels the connection between the clip 12B and the clip 12C at the same time as the completion of the clamping of the clip 12B by the connection ring 14B, and the clipping by the clip 12B is completed.

Regarding the clips 12C, 12D, and 12E, the clipping is performed in the same way as in the clip 12B.

After the clipping of the rearmost clip 12E is completed, i.e., after all the clips 12 are used up, the claw 76 of the sheath manipulating handle 54 is moved from the sixth notch 66f to the seventh notch 66g. More specifically, by moving the claw 76 of the sheath manipulating handle 54 by a predetermined interval (predetermined distance) N, the sheath 16 is moved to the manipulating portion side by a predetermined distance N, and the dummy clip 18 is protruded from the forward end of the sheath 16, whereby the dummy clip 18 is removed from the manipulating wire 20.

The predetermined interval N refers to the distance by which the sheath 16 is moved to the manipulating portion side so that the dummy clip 18 is protruded from the forward end of the sheath 16 to be placed in a removable position after all the clips 12 are used up.

For example, usually, in the state in which all the clips 12 are used up, the forward end of the dummy clip 18 is substantially matched with the forward end of the sheath 16. Therefore, the movement distance of the sheath 16 required for moving the dummy clip 18 to the removable position may be determined from this state.

As described above, in Embodiment 1, by setting the distance from the first notch 66a to the second notch 66b to be a predetermined value K obtained by adding the sinking amount of the foremost clip 12A with respect to the sheath 16 to the load interval L of the clips 12, even in the case where the foremost clip 12A sinks inside the sheath 16, the foremost clip 12A can be placed in a usable state by moving the claw 76 of the sheath manipulating handle 54 from the first notch 66a to the second notch 66b.

It is preferred to fix the sheath 16 by pushing out the lever 60 to the forward end side in pushing out the manipulating wire 20 to the forward end side.

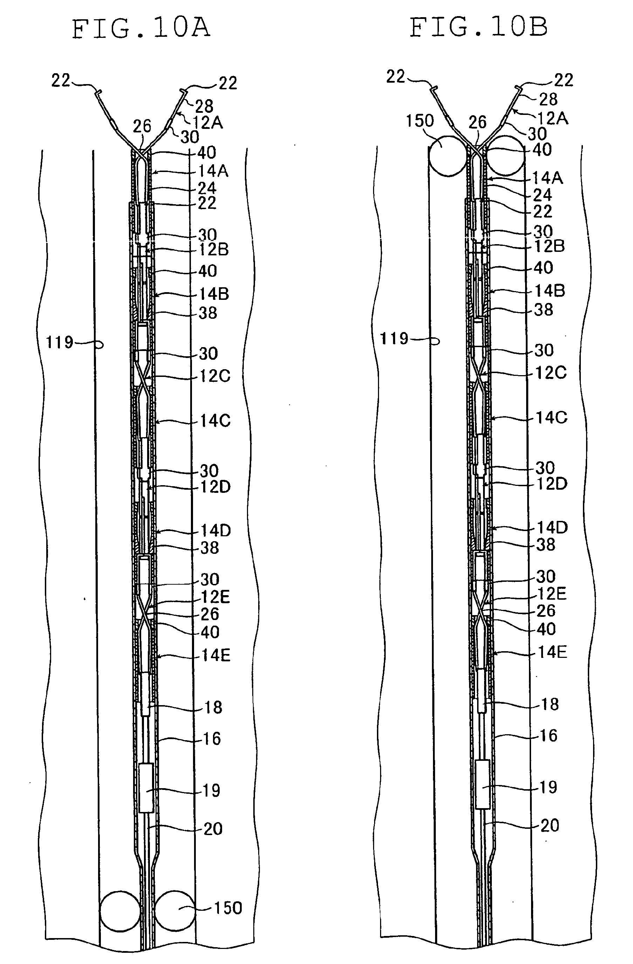

For example, as illustrated in FIG. 10A, a balloon 150 may be provided between a forceps channel 119 of the endoscope and the sheath 16 of a portion not retaining the connection clip to fix the sheath 16 at a predetermined position of the forceps channel 119.

Further, as illustrated in FIG. 10B, the balloon 150 may be provided similarly between the forceps channel 119 of the endoscope and the forward end portion of the sheath 16 to fix the sheath 16 at a predetermined position of the forceps channel 119.

It is assumed that the balloon 150 swells only when necessary.

### Embodiment 2

Next, Embodiment 2 of the present invention is described. In Embodiment 1, in order to cause the foremost clip 12A to protrude from the sheath 16, the sheath 16 is pulled to the operator's hand side by the sheath manipulating handle 54 of the manipulating portion 50 to allow the sheath 16 to retreat with respect to the clips 12. Further, when the clipping is performed by each of the clips 12, the manipulating wire 20 connected to the clips 12 is pulled to the operator's hand side by the lever 60 of the manipulating portion 50 to allow the clips 12 to advance or retreat with respect to the sheath 16. In contrast, in Embodiment 2, the sheath 16 is fixed, and only the advancement and retreat of the manipulating wire 20 causes the clips 12 to protrude from the sheath 16 and the clipping to be performed.

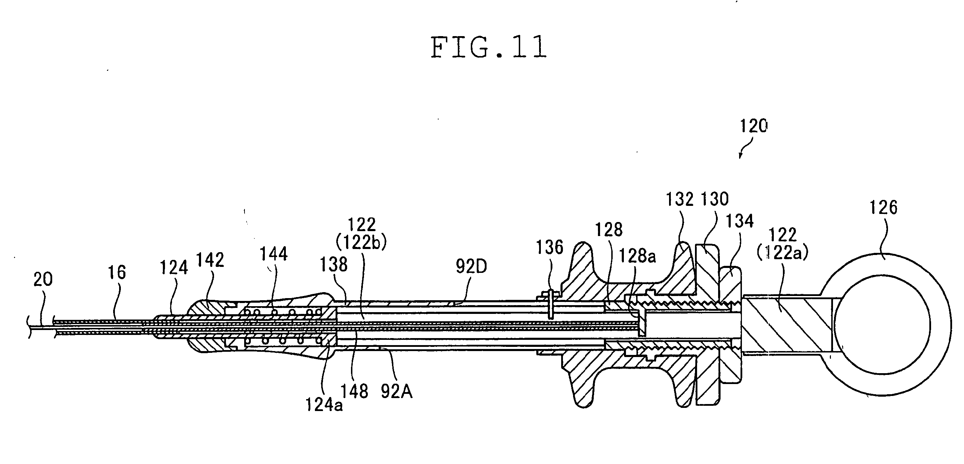

FIG. 11 is a cross-sectional view illustrating a configuration of a manipulating portion 120 used in a successive clipping device according to Embodiment 2. The manipulating portion 120 can be used for a three successive clipping device, for example. In the case where three clips 12 are loaded for three successive clipping manipulations in the manipulation operating portion 11 of the clipping device 10 according to Embodiment 1, the manipulating portion 120 can be used in place of the manipulating portion 50. Even in the case where the manipulating portion 120 is used, the relative positional relationship between the respective portions of a clip loading portion (forward end portion of sheath 16) in the clipping and the like is the same as that in Embodiment 1.

The left side of the manipulating portion 120 in the figure is connected to the clip 12 and the sheath 16, and the right side thereof is manipulated by an operator. In the following description, the left end in FIG. 11 is referred to as a forward end and the right end therein is referred to as a proximal end.

The manipulating portion 120 includes a main body rail 122 forming the center, a forward end member 124 fixed to the forward end of the main body rail 122, a finger hook ring 126 attached to the proximal end of the main body rail 122, a wire fixing member 128, an adjustment dial 130, a slider 132, and a lock dial 134 provided outside of the main body rail 122, and a slider guide 138 and a position regulating member 142 provided outside of the forward end member 124.

The proximal end of the sheath 16 is retained at the forward end of the forward end member 124, and the proximal end of the manipulating wire 20 is retained at a wire connecting portion 128a of the wire fixing member 128. For example, the operator hooks the thumb on the finger hook ring 126, and hooks the forefinger and the middle finger on the slider 132, thereby allowing the slider 132 to slide in an advancement or retreat direction with respect to the finger hook ring 126 in a slide manner. When the slider 132 moves, the wire fixing member 128 also moves, and the manipulating wire 20 retained at the wire connecting portion 128a also moves. On the other hand, the sheath 16 is connected to the finger hook ring 126 via the forward end member 124 and the main body rail 122. Thus, the manipulating wire 20 is allowed to advance or retreat with respect to the sheath 16 by the movement of the slider 132 with respect to the finger hook ring 126. Hereinafter, the configuration of each portion of the manipulating portion 120 is described in detail.

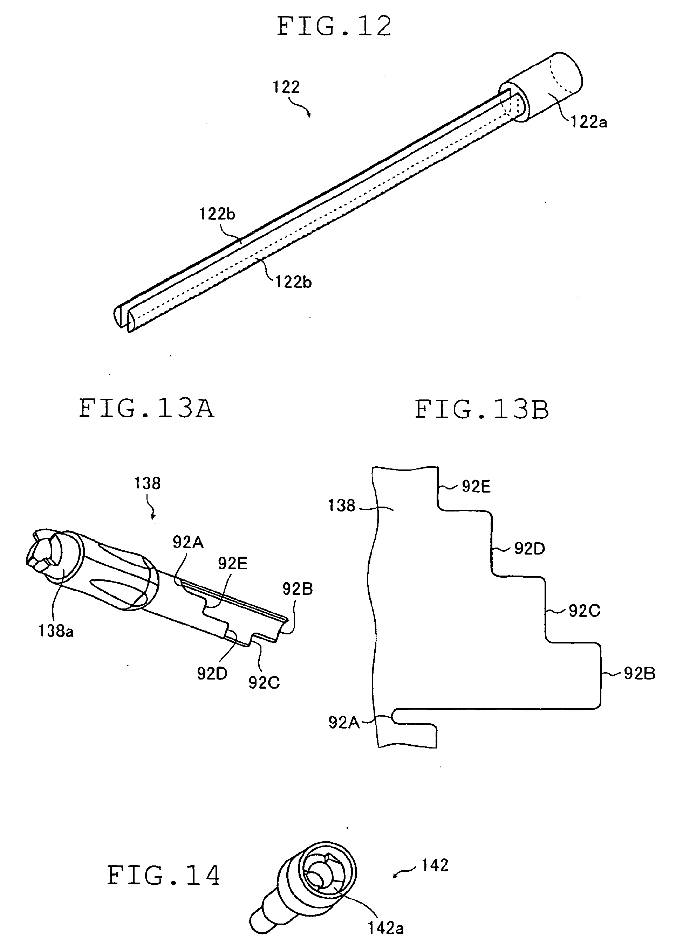

As illustrated in FIG. 12, the main body rail 122 has a shape in which two bar members 122b, 122b having a substantially semi-circular shape in cross-section extend from a cylindrical proximal end portion 122a. The two bar members 122b, 122b are placed at a predetermined distance therebetween with plane portions opposed to each other, and curved surfaces of both the members form one cylindrical surface. In other words, the bar members 122b, 122b have a shape in which the center of a cylinder which is coaxial with and is thinner than the proximal end portion 122a is cut off with a predetermined width along the axial line. In the main body rail 122 illustrated in FIG. 11, the bar member 122b on the front side in the figure is removed, and the plane portion of the bar member 122b on the back side is displayed.

The interval between the two bar members 122b, 122b is set to be such that the forward end portions of the wire connecting portion 128a of the wire fixing member 128 and the slider pin 136 to be attached to the slider 132 can be inserted. As illustrated in FIG. 11, the forward ends of the wire connecting portion 128a and the slider pin 136 are inserted between the two bar members 122b, 122b, and guided by the bar members 122b, 122b to move in the extension direction of the main body rail 122. More specifically, in the main body rail 122, the bar members 122b, 122b function as a rail for the wire connecting portion 128a (wire fixing member 128) and the slider pin 136 (slider 132).

The forward end member 124 is a cylindrical component having a flange-shaped portion 124a with a large diameter at the proximal end portion. The forward end of the main body rail 122 is fixed to the end surface of the proximal end of the forward end member 124. Further, the proximal end portion of the sheath 16 is inserted to be retained at the forward end of the forward end member 124. The manipulating wire 20 extends from the proximal end of the sheath 16 to pass through an inner space of the forward end member 124. The outer diameter of the cylindrical portion of the forward end member 124 is substantially equal to the inner diameter of the forward end portion of the slider guide 138 placed outside of the forward end member 124, and the outer diameter of the flange-shaped portion 124a is substantially equal to the inner diameter of the slider guide 138 on the rear end side. This enables the slider guide 138 to slide with respect to the forward end member 124.

The finger hook ring 126 is a component having a ring portion into which the operator can insert the finger, and is attached to the cylindrical proximal end portion 122a of the main body rail 122.

The wire fixing member 128 is a cylindrical member having an inner diameter substantially equal to the outer diameters of the two bar members 122b, 122b of the main body rail 122. The outer circumferential surface of the proximal end portion of the wire fixing member 128 is threaded. The wire connecting portion 128a protruding to the inner surface side is formed in a substantially central portion of the wire fixing member 128 in the axial direction. The proximal end of the manipulating wire 20 extending through the forward end member 124 is fixed at a position matched with the central axis of the wire fixing member 128 (i.e., the central axis of the manipulating portion 120) of the wire connecting portion 128a. The manipulating wire 20 is covered with a reinforcement tube 148 in a range from the inside of the forward end of the sheath 16 to the wire connecting portion 128a and reinforced so that the manipulating wire 20 does not break inside the manipulating portion 120.

The adjustment dial 130 is a cylindrical component with a flange fitted to the outer side of the wire fixing member 128. The inner circumferential surface of the adjustment dial 130 on the proximal end side is threaded, and this threaded portion is fitted to the threaded portion of the wire fixing member 128. When the flange portion of the adjustment dial 130 is rotated by the operator, the adjustment dial 130 advances or retreats along the thread of the wire fixing member 128 in accordance with the rotation direction. On the outer circumferential surface of a thin portion of the adjustment dial 130, a projection to be engaged with a recession of the slider 132 is formed around the entire circumference.

The slider 132 has a bobbin shape, and the operator can hook the finger onto the slider 132 to move it easily. The wire fixing member 128 is inserted into the slider 132. Further, the proximal end side portion of the slider 132 has a larger inner diameter, into which the adjustment dial 130 is inserted from the proximal end side. Over the entire inner circumferential surface of the slider 132, a recession to be engaged with the projection of the adjustment dial 130 is formed. Due to the engagement between the recession and the projection, the slider 132 moves integrally with the adjustment dial 130 in the axial direction, while the adjustment dial 130 and the slider 132 can be rotated freely in the circumferential direction.

The lock dial 134 is a ring-shaped component with the inner circumferential surface threaded, corresponding to the thread of the proximal end portion of the wire fixing member 128. A screw is tightened until the lock dial 134 comes into contact with the end surface of the proximal end of the adjustment dial 130 after the position of the adjustment dial 130 is adjusted, whereby the position of the adjustment dial 130 after the adjustment is locked.

The slider 132 and the adjustment dial 130 are engaged with each other via the recession and the projection, and the adjustment dial 130 and the lock dial 134 are engaged with the wire fixing member 128 at the threaded portion. Therefore, when the slider 132 is moved in an advancement or retreat direction (right and left direction in the figure), those four components move integrally.

The slider pin 136 is inserted into the forward end portion of the slider 132 from the outer side to the inner side to be fixed. The forward end of the slider pin 136 reaches an area between the bar members 122b, 122b of the main body rail 122, and can move between the two bar members 122b, 122b.

The slider guide 138 is a substantially cylindrical member provided outside of the forward end member 124. As described above, the inner diameter of the forward end portion of the slider guide 138 is substantially equal to the outer diameter of the tubular portion of the forward end member 124. The inner diameter of the slider guide 138 on the proximal end side is substantially equal to the outer diameter of the flange-shaped portion 124a of the forward end member 124. The slider guide 138 is slidably supported by the flange-shaped portion 124a. Further, the outer diameter of the proximal end side portion of the slider guide 138 is slightly smaller than the inner diameter of the slider 132, and when the slider 132 moves to the forward end side, the slider guide 138 can enter the inner side of the slider 132. The slider guide 138 is rotated against the position regulating member 142 at the forward end by the manipulation of the operator; therefore, an inclined surface is formed on the outer surface so that the operator can hold it easily.

A coil spring 144 is placed with respect to the forward end member 124 between the flange-shaped portion 124a of the forward end member 124 and the inner surface of the slider guide 138. The coil spring 144 is a compression spring, which biases the slider guide 138 to the forward end side against the forward end member 124 (flange-shaped portion 124a) that is a fixing member, thereby pressing the slider guide 138 toward the position regulating member 142. The position regulating member 142 is fixed to the forward end member 124.

With reference to FIGS. 13A, 13B, and 14, the slider guide (slider movement amount regulating member) 138 and the position regulating member 142 are described in more detail. FIG. 13A is a perspective view of the slider guide 138, and FIG. 13B is a developed view of a stepped portion on the proximal end side of the slider guide 138. FIG. 14 is a perspective view of the position regulating member 142.

As illustrated in FIG. 13A, on the end surface of a connection portion 138a with the position regulating member 142 in the forward end portion of the slider guide 138, four projections in a sawtooth shape with inclination angles of two sides being different are formed in the circumferential direction at an interval of 90°. The projection in a sawtooth shape has one surface with a gentle inclination angle and the other surface with an inclination angle of a substantially right angle. Further, as illustrated in FIG. 14, on a connection portion 142a with the slider guide 138 in the rear end portion of the position regulating member 142, four projections similar to those on the connection portion 138a of the slider guide 138 are provided.

The slider guide 138 is pressed against the position regulating member 142 by the coil spring 144 while being engaged with the position regulating member 142. Therefore, unless the external force from the operator is not applied, the slider guide 138 does not rotate with respect to the position regulating member 142. Further, the position regulating member 142 and the slider guide 138 are engaged with each other via sawtooth-shaped unevenness; therefore, when the operator tries to rotate the slider guide 138 axially, the slider guide 138 rotates in a direction in which steep surfaces (with an inclination angle of a substantially right angle) of the projections of the connection portions 138a and 142a leave each other but does not rotate in a direction in which the steep surfaces abut each other. In the illustrated example, the slider guide 138 can rotate counterclockwise with respect to the proximal end side (right side in FIG. 13A) but cannot rotate clockwise.

When the gentle surface of the connection portion 138a of the slider guide 138 rotates along with the gentle surface of the connection portion 142a of the position regulating member 142 at an angle of 90° to overpass the apexes, they are engaged with each other via the next unevenness. Thus, the slider guide 138 rotates by 90°.

As illustrated in FIGS. 13A and 13B, in the cylindrical portion of the slider guide 138 on the proximal end side, five sides 92 (sides 92A, 92B, 92C, 92D, 92E) are formed, which are parallel to the surface orthogonal to the central axis and placed at different positions in the central axis direction. More specifically, as illustrated in FIG. 13A, the cylindrical portion of the slider guide 138 on the proximal end side has a stepped portion in the central axis direction.

Among the five sides 92, the side 92A is positioned closest to the sheath forward end side, and the sides 92E, 92D, 92C, and 92B are placed closer to the finger hook ring 126 side in this order. Further, the five sides 92 are formed in the order of the sides 92A, 92B, 92C, 92D, and 92E in this order in the rotation direction, and the sides 92E and 92A are adjacent to each other. Further, the lower ends of the sides 92A, 92C, 92D, and 92E in FIG. 13B are apart from each other by about 90°.

In FIG. 11, the side 92A appears on the lower side of the figure, and the side 92D appears on the upper side.

The stepped portion formed by adjacent two sides among the five sides 92 (sides 92A, 92B, 92C, 92D, 92E) acts as a guide groove of the slider pin 136. Every time the slider guide 138 rotates by 90°, any of the stepped portion formed by the adjacent two sides is placed so as to be matched with the rail position of the main body rail 122, i.e., the position between the two bar members 122b, 122b. Thus, when the slider 132 is moved in an advancement or retreat direction by the manipulation of the operator, the forward end portion of the slider pin 136 is guided to the main body rail 122 and at the same time is guided to any of the stepped portion formed by the two adjacent sides. The movement amount of the slider 132 is determined by the height of the stepped portion (length in the axial direction) formed by the adjacent two sides among the five sides 92 (sides. 92A, 92B, 92C, 92D, 92E).

More specifically, the slider guide 138 regulates the movement limit to the forward end side of the slider pin 136 moving along the main body rail 122 by the stepped portion formed by the adjacent two sides among the five sides 92, thereby regulating the movement positions of the slider 132, and the adjustment dial 130, the wire fixing member 128, and the lock dial 134 moving together with the slider 132, and regulating the movement position of the manipulating wire 20 connected to the wire fixing member 128.

The movement limit of the slider 132 and the like on the proximal end side is regulated by the position at which the end surface of the lock dial 134 on the proximal end side abuts the end surface of the proximal end portion 122a of the main body rail 122 on the forward end side.

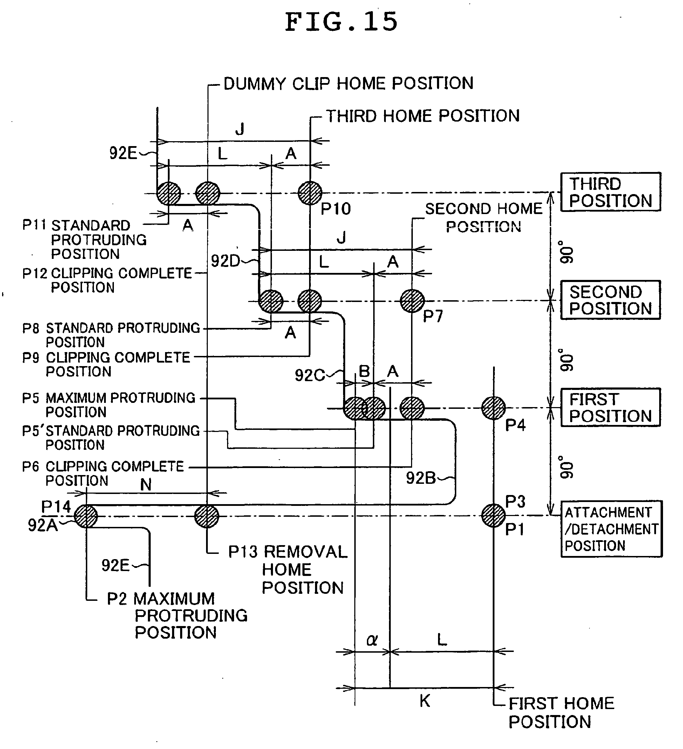

Referring to FIG. 15, the function of the manipulating portion 120 is described.

FIG. 15 is a developed view of stepped portions on the proximal end side of the slider guide 138, illustrating the positional relationship between the slider guide 138 and the slider pin 136 during the clipping. The slider 132 or the like places the position of the slider pin 136, which is in the movement limit on the proximal end side, at a home position P1. The home position of the slider 132 corresponds to the position where the slider 132 is pulled most to the proximal end side, and is defined when the lock dial 134 abuts the proximal end portion 122a of the main body rail 122. When the slider pin 136 is placed at the home position P1, the forward end of the manipulating wire 20 is retreated from the forward end of the sheath 16 by the amounts of the three clips 12, the dummy clip 18, and the connecting member 19.

First, the slider guide 138 is rotated to set the slider pin 136 at a position matched with the stepped portion formed by the side 92A and the side 92E. At this time, the slider pin 136 is placed at the home position P1 illustrated in FIG. 15. When the slider 132 is moved forwards to move the slider pin 136 to a maximum protruding position P2 of the side 92A from the home position P1, the manipulating wire 20 moves to the forward end side by the length from the home position P1 to the side 92A. This causes the forward end of the manipulating wire 20 to protrude from the forward end of the sheath 16 to allow the connecting member 19 to be attached to the manipulating wire 20.

In Embodiment 2, the length from the home position P1 to the side 92A is defined as the distance by which the forward end of the manipulating wire 20 protrudes from the forward end of the sheath 16 to allow the connecting member 19 to be attached to the manipulating wire 20.

When the slider 132 is moved rearwards to return the slider pin 136 to a position P3 that is the same position as the home position P1 after the connecting member 19 is attached to the manipulating wire 20, the manipulating wire 20 is pulled in the sheath 16, whereby the connecting member 19 connected to the forward end of the manipulating wire 20, the dummy clip 18, and the subsequent three clips 12 are pulled in the sheath 16. Thus, the loading of the clips in the sheath 16 is completed.

Next, the slider guide 138 is rotated by 90°. As a result, the slider pin 136 is placed at a home position P4 of a first clip (hereinafter, which may be referred to merely as the first home position P4) which is equal to the home positions P1 and P3 in the axial direction and is different therefrom by 90° in the circumferential direction, and the slide pin 136 is matched with the stepped portion formed by the sides 92B and 92C. When the slider 132 is moved forwards to move the slider pin 136 from the first home position P4 to a maximum protruding position P5 at which the slider pin 136 comes into contact with the side 92C, the manipulating wire 20 and the clips and the like connected thereto move to the forward end side by the length (predetermined extrusion length K) from the first home position P4 to the side 92C.

The length from the first home position P4 to the side 92C, i.e., the predetermined extrusion length K is described later.

When the slider pin 136 is moved by the predetermined extrusion length K and the manipulating wire is extruded to the forward end side by the predetermined extrusion length K as described above, in most cases, a gap is formed between the lower end of a skirt portion of the connection ring and the forward end of the sheath (see FIG. 9B). Therefore, the slider pin 136 is moved from the maximum protruding position P5 to a standard protruding position P5', and the manipulating wire is moved to the proximal end side by the distance between the maximum protruding position P5 and the standard protruding position P5', whereby the gap between the lower end of the skirt portion and the forward end of the sheath thus formed is buried.

Here, the distance between the maximum protruding position P5 and the standard protruding position P5' is preferably set to be a value corresponding to the gap formed between the lower end of the skirt portion of the connection ring protruding from the forward end of the sheath and the forward end of the sheath.

Then, when the slider 132 is moved rearwards, and the slider pin 136 is moved to a clipping complete position P6, the clamping of the foremost (first) clip with the connection ring and the detachment from the next clip are performed in the manipulation operating portion at the forward end of the sheath 16. Thus, the first clipping is completed.

More specifically, a distance A between the standard protruding position P5' and the clipping complete position P6 corresponds to the distance by which the first clip 12, i.e., the slider 132 is moved rearwards when clipping is performed with the clip 12 placed in a state capable of performing manipulation.

Then, for performing the next clipping, the slider guide 138 is rotated in the same direction as mentioned above by 90°. This places the slider pin 136 at a home position P7 of a second clip (hereinafter, which may be referred to merely as a second home position P7) which is equal to the clipping complete position P6 in the axial direction and is different therefrom by 90° in the circumferential direction, and the slide pin 136 is matched with the stepped portion formed by the sides 92C and 92D. When the slider 132 is moved forwards to move the slider pin 136 from the second home position P7 to the side 92D, i.e., the standard protruding position P8, the manipulating wire 20 and the clips and the like connected thereto move to the forward end side by the length (predetermined extrusion length J) from the second home position P7 to the standard protrusion position P8.

The length from the second home position P7 to the side 92D, i.e., the predetermined extrusion length J is described later.

Then, when the slider 132 is moved rearwards, and the slider pin 136 is moved to a clipping complete position P9, the clamping of the second clip 12 with the connection ring 14 and the detachment from the next clip 12 are performed in the manipulation operating portion at the forward end of the sheath 16. Thus, the second clipping of the clip 12 is completed.

Then, for performing the next clipping, the slider guide 138 is rotated in the same direction as mentioned above by 90°. This places the slider pin 136 at a home position P10 of a third clip (hereinafter, which may be referred to merely as a third home position P10) which is equal to the clipping complete position P9 in the axial direction and is different therefrom by 90° in the circumferential direction, and the slide pin 136 is matched with the stepped portion formed by the sides 92D and 92E. When the slider 132 is moved forwards to move the slider pin 136 from the third home position P10 to the side 92E, i.e., the standard protruding position P11, the manipulating wire 20 and the clips and the like connected thereto move to the forward end side by the length (predetermined extrusion length J) from the third home position P10 to the standard protrusion position P11.

Then, when the slider 132 is moved rearwards, and the slider pin 136 is moved to a clipping complete position P12, the clamping of the third clip with the connection ring and the detachment from the dummy clip are performed in the manipulation operating portion at the forward end of the sheath 16. Thus, the third clipping is completed.

Finally, in order to remove the dummy clip 18 from the sheath 16, the slider guide 138 is rotated in the same direction as mentioned above by 90°. As a result, the slider pin 136 is placed at a home position P13 of a dummy clip removal (hereinafter, which may be referred to merely as the removal home position P13) which is equal to the clipping complete position P12 in the axial direction and is different therefrom by 90° in the circumferential direction, and the slide pin 136 is matched with the stepped portion formed by the sides 92E and 92A. When the slider 132 is moved forwards to move the slider pin 136 from the removal home position P13 to the side 92A, i.e., the maximum protruding position P14, the manipulating wire 20 and the clips and the like connected thereto move to the forward end side by the length (predetermined extrusion length N) from the removal home position P13 to the maximum protruding position P14.

The length from the removal home position P13 to the maximum protruding position P14, i.e., the predetermined extrusion length N refers to the length that is required for moving the dummy clip at a time when the third clipping is completed is moved to the forward end side and the dummy clip is allowed to protrude from the sheath 16.

In the case where the manipulating wire 20 is stretched during the use for a long period of time, the following adjustment is conducted. First, the lock dial 134 is released, and the adjustment dial 130 is rotated to move the adjustment dial 130 to the forward end side with respect to the wire fixing member 128. The slider 132 is engaged with the projection of the adjustment dial 130, therefore the adjustment dial 130 is moved together with the slider 132 in the axial direction. It should be noted that, because the adjustment dial 130 and the slider 132 move freely in the rotation direction, only the adjustment dial 130 can be rotated with respect to the slider 132 whose movement in the circumferential direction is regulated by the slider pin 136. When the adjustment dial 130 is moved to such a degree that the flextion of the manipulating wire 20 is eliminated, the lock dial 134 is fastened again to lock the positions of the adjustment dial 130 and the slider 132.

The home positions P1 and P4 of the slider 132 are defined when the lock dial 134 abuts the proximal end portion 122a of the main body rail 122. Therefore, the position of the slider 132 with respect to the main body rail 122 at the home positions P1 and P4 remain unchanged. On the other hand, when the adjustment dial 130 and the slider 132 are moved to the forward end side with respect to the wire fixing member 128, the wire fixing member 128 is moved rearwards relative to the slider 132, and the wire connecting portion 128a is moved similarly. Thus, the proximal end of the manipulating wire 20 at the home position is moved rearwards, whereby the flexion caused by the extended amount of the manipulating wire 20 can be eliminated.

The predetermined extrusion length K is defined as a value obtained by adding the previously calculated sinking amount α of the foremost clip 12A with respect to the sheath 16 to the load interval L of the clips 12, in the same way as in the predetermined pulling length K in Embodiment 1. If the sinking of the first clip is eliminated by the predetermined extrusion length K, the sinking of the second and subsequent clips can be eliminated.

Next, the predetermined extrusion length J in the present embodiment is described.

The length from the second home position P7 to the standard protruding position P8, and the length from the third home position P10 to the standard protruding position P11 are both set to be the predetermined extrusion length J. The predetermined extrusion length J is the length obtained by adding the load interval L of the adjacent clips in the sheath 16 to the interval A between the standard protruding position P5' and the clipping complete position P6 or the interval A between the standard protruding position P8 and the clipping complete position P9.

The slider pin 136 is placed at the home position P7 immediately after the completion of the manipulation of the first clip. Therefore, the second and the subsequent clips are placed at a position moved toward the proximal end side by the distance A from the position where the lower end of the skirt portion of the connection ring corresponding to the first clip is matched with the upper end of the sheath 16. More specifically, the second clip is placed at a position moved toward the proximal end side by the distance A from the position where the forward end of the second clip is matched with the forward end of the sheath 16.

Therefore, in order to place the second clip in a usable state, first, it is necessary to extrude the slider pin 136 to the forward end side by the distance A so as to move the second clip to the position where the forward end of the second clip is matched with the forward end of the sheath 16. Further, in order for the skirt portion of the connection ring corresponding to the second clip to place the second clip in a usable state from the above-mentioned state, it is necessary to extrude the slider pin 136 by the load interval L of the clips.

As described above, the predetermined extrusion length J from the second home position P7 to the standard protruding position P8 is set to be a value obtained by adding the distance (distance by which the manipulating wire is pulled toward the proximal end side during the manipulation by a clip in a standby state) A between the standard protruding position P5' and the clipping complete position P6 to the load interval L of the clips in the sheath 16.

Therefore, when the slider pin 136 is moved from the second home position P7 to the standard protruding position P8, the second clip 12 is placed in a usable state.

Similarly, the predetermined extrusion length J from the third home position P10 to the standard protruding position P11 is set to be a value obtained by adding the distance A between the standard protruding position P11 and the clipping complete position P12 to the load interval L of the clips in the sheath 16.

### Embodiment 3

According to Embodiment 1 described above, the interval between the first notch 66a and the second notch 66b of the manipulating portion 50 is set to be a predetermined value K obtained by adding the sinking amount of the foremost clip 12A with respect to the sheath 16 to the load interval L of the clips 12, and each interval between the adjacent notches 66 from the second notch 66b to the sixth notch 66f is set to be a value L equal to the load interval of the clips 12. However, if the foremost clip 12A can be placed in a usable state by inserting the sheath 16 loaded with the clips into the forceps channel of the endoscope, allowing the sheath 16 to reach the forward end portion of the endoscope, moving the claw 76 of the manipulating portion 50 from the first notch 66a to the second notch 66b, pulling the sheath 16 by the load interval L of the clips 12, and moving the sheath 16 to the front and the back of the lever 60, the interval between the first notch 66a and the second notch 66b can also be set to be a value L equal to the load interval of the clips 12.

### Embodiment 4

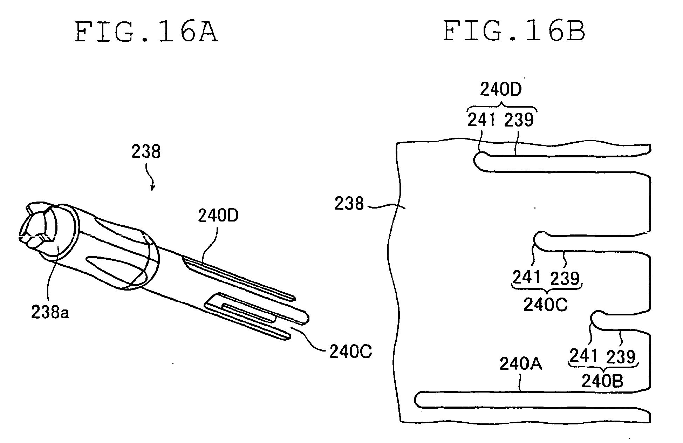

In Embodiment 4, a slider guide 238 illustrated in FIG.16A is used instead of the slider guide 138 of the manipulating portion 120 according to Embodiment 2.

A connecting portion 238a is formed on the forward end side of the slider guide 238 and a cylindrical portion is formed on the proximal end side. The connecting portion 238a has four projections that are engaged with four projections of the position regulating member 142 in the same way as in the connecting portion 138a of the slider guide 138 in Embodiment 2.

In the cylindrical portion on the proximal end side of the slider guide 238, four slider guide grooves 240A, 240B, 240C, and 240D extending from the end surface on the proximal end side along the rotation shaft are formed at an interval of 90°. As illustrated in FIG. 16B, the slider guide grooves 240A to 240D have different groove lengths, and only the slider guide groove 240A is different from the others in shape. In the illustrated example, the slider guide groove 240A is longest, and the other slider guide grooves become short in the order of the slider guide groove 240D, the slider guide groove 240C, and the slider guide groove 240B. Further, the slider guide groove 240A extends linearly in the extension direction of the slider guide 238, and the slider guide grooves 240B to 240D are respectively composed of a groove extending linearly in the extension direction (hereinafter, which may be referred to as a linear portion 239) and a fine-adjustment groove (hereinafter, which may be referred to as a curved portion 241) having a shape curved in the rotation direction of the slider guide 238 from the forward end of the linear portion, i.e., a shape curved at a predetermined angle in the rotation direction with respect to the linear portion.

The slider guide grooves 240A to 240D have a function as the guide grooves of the slider pin 136, and the widths of the grooves are substantially equal to the diameter of the slider pin 136. Every time the slider guide 238 rotates by 90°, any of the slider guide grooves 240A to 240D is placed so as to be matched with a rail position of the main body rail 122 illustrated in FIG. 12, i.e., a position between two bar members 122b, 122b. Thus, when the slider 132 is advanced or retreated by the manipulation of the operator, the forward end portion of the slider pin 136 is guided to the main body rail 122, and simultaneously, the intermediate portion is guided to any of the slider guide grooves 240A to 240D. The movement amount of the slider 132 is determined by the length of the slider guide grooves 240A to 240D in the direction of the slider guide 238.

More specifically, the slider guide 238 defines the movement limit to the forward end side of the slider pin 136 that moves along the main body rail 122 by the slider guide grooves 240A to 240D, thereby defining the movement positions of the slider 132, and the adjustment dial 130, the wire fixing member 128, and the lock dial 134 that move along with the slider 132 to define the movement position of the manipulating wire 20 connected to the wire fixing member 128.

The length of the linear portion 239 of the slider guide groove 240B of the slider guide 238 is matched with the movement amount of the manipulating wire 20 until the foremost clip 12A protrudes from the forward end of the sheath 16 and the skirt portion 38 of the connection ring 14 is opened. The length of the linear portion 239 of the slider guide groove 240C is matched with the movement amount of the manipulating wire 20 until the second clip 12B protrudes from the forward end of the sheath 16, and the skirt portion 38 of the connection ring 14 is opened. The length of the linear portion 239 of the slider guide groove 240D is matched with the movement amount of the manipulating wire 20 until the third clip 12C protrudes from the forward end of the sheath 16 and the skirt portion 38 of the connection ring 14 is opened.

Thus, the clip 12 can be placed in a usable state (standby state) by adjusting the slider pin 136 with the slider guide grooves 240B to 240D corresponding to the clip 12 to be protruded and moving the slider pin 136 by the length of the linear portion 239 of the slider guide grooves 240B to 240D.

However, in some cases, even when the slider pin 136 is adjusted with the slider guide grooves 240B to 240D corresponding to the clip 12 to be protruded, and the slider pin 136 is moved by the length of the linear portion 239 of the slider guide grooves 240B to 240D, the clip 12 cannot be placed in a usable state and clipping cannot be performed.

The forward end portions of the slider guide grooves 240B to 240D to be used for protruding the clip 12 are respectively curved in the rotation direction of the slider guide 238. More specifically, curved portions (fine-adjustment grooves) 241 are formed respectively in the forward end portions of the slider guide grooves 240B to 240D.

Therefore, the slider pin 136 is adjusted with the slider guide grooves 240B to 240D corresponding to the clip 12 to be protruded, and the slider pin 136 is moved through the linear portion 239 of the slider guide grooves 240B to 240D, and thereafter, the slider pin 136 can be moved while rotating in the curved direction.

The distance in the extension direction of the slider guide 238 of the curved portion 241 of the slider guide grooves 240B to 240D is preferably smaller than the distance by which the slider 132 is pulled to the proximal end side when clipping is performed, and is particularly preferably more than 0 mm to less than 0.1 mm.

As described above, by providing the curved portion 241 at each forward end of the slider guide grooves 240B to 240D to be used for allowing the clip 12 to protrude, the manipulating wire 20 can be extruded slightly to the forward end side, i.e., can be finely adjusted in the case where the clip 12 does not protrude sufficiently from the sheath 16 and the skirt 38 is not opened merely by moving the slider pin 136 only through the linear portion 239 of the slider guide grooves 240B to 240D.

As described above, the fine adjustment for allowing the clip 12 to protrude is conducted by moving the slider pin 136 while placing the slider pin 136 in the curved portion 241 of the slider guide grooves 240B to 240D. The operation of moving the slider pin 136 while placing the slider pin 136 in the curved portion 241 of the slider guide grooves 240B to 240D requires the operator to perform operations in different directions, which can prevent a malfunction.

Further, the slider pin 136 is moved while being placed in the curved portion 241 of the slider guide grooves 240B to 240D, and therefore the manipulating wire 20 can be provided with a twist. Therefore, compared with the case of extruding the manipulating wire 20 only in the extension direction of the slider guide 238, the skirt portion 38 of the clip 12 is placed in a state capable of being opened easily.

Next, the function of the successive clipping device in Embodiment 4 is described.

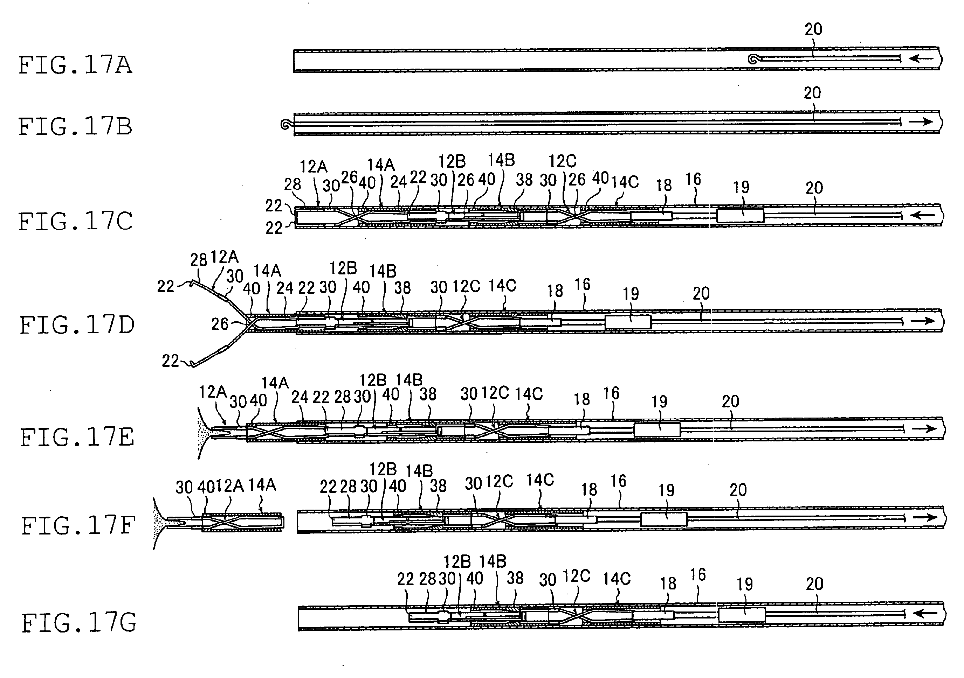

FIGS. 17A to 17G are partial cross-sectional views illustrating a state in stages during the clipping of the clipping device 10 of the present invention.

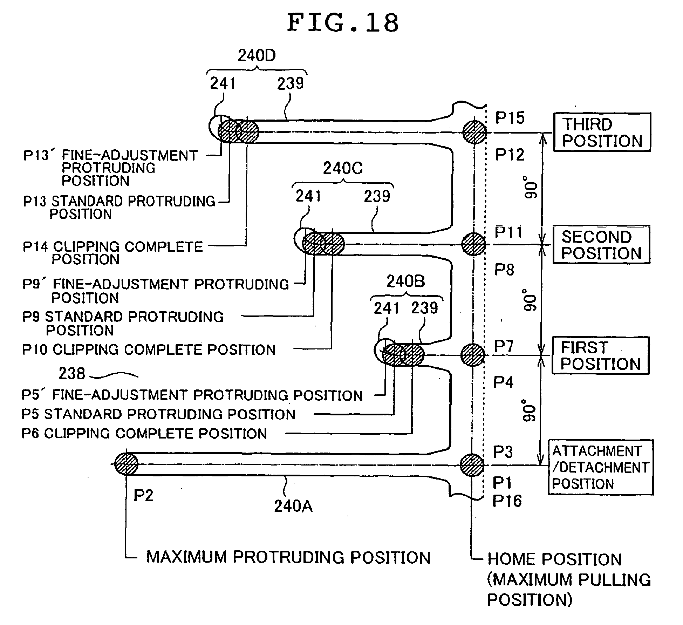

Further, FIG. 18 is a developed view of the portions of the slider guide grooves 240A to 240D, illustrating the positional relationship between the slider guide 238 and the slider pin 136 during the clipping.

In FIG. 18, it is assumed that the position of the slider pin 136 when the slider 132 or the like is at the movement limit on the proximal end side is the home position P1. The home position of the slider 132 corresponds to the position at which the slider 132 is pulled to the proximal end side at maximum, and is defined when the lock dial 134 abuts the proximal end portion 122a of the main body rail 122. When the slider pin 136 is at the home position P1, the forward end of the manipulating wire 20 is at a position retreated from the forward end of the sheath 16 by three clips 12, the dummy clip 18, and the connecting member 19.

Further, assuming that the position of the slider pin 136 at which the clip 12 protrudes from the sheath 16 and is generally supposed to be placed in a state capable of performing manipulation (standby state) is the standard protruding position, and the position of the slider pin at which the clip 12 protrudes from the sheath 16 further compared with the case where the slider pin 136 is at the standard protruding position is the fine-adjustment protruding position, the portion from the end on the home position side to the standard protruding position of the slider guide grooves 240B to 240D of the slider guide 238 described above is the linear portion 239, and the portion from the standard protruding position to the fine-adjustment protruding position is the curved portion 241 described above, respectively.

For using the successive clipping device 10, first, the slider guide 238 in the manipulating portion 120 is rotated to a position at which the slider guide groove 240A is matched with the slider pin 136. Then, as illustrated in FIG. 17A, when the slider 132 is moved forwards to move the slider pin 136 to the maximum protruding position P2 at the forward end of the slider guide groove 240A, the manipulating wire 20 is moved to the forward end side by the length of the slider guide groove 240A, and as illustrated in FIG. 17B, the forward end of the manipulating wire 20 protrudes from the forward end of the sheath 16, which makes it possible to attach the connecting member 19 to the manipulating wire 20. After the connecting member 19 is attached to the manipulating wire 20, the slider 132 is moved rearwards to return the slider pin 136 to P3 that is the same position as the home position P1, the manipulating wire 20 is pulled in the sheath 16, whereby the connecting member 19, the dummy clip 18, and the subsequent three clips 12 connected to the forward end of the manipulating wire 20 are pulled in the sheath 16. Thus, as illustrated in FIG. 17C, the foremost clip 12A is matched with the forward end of the sheath 16, and the loading of the clips in the sheath 16 is completed.

Next, the slider guide 238 is rotated by 90°. As a result, the slider pin 136 is placed at the position P4 which is equal to the home positions P1 and P3 in the axial direction and is different therefrom by 90° in the circumferential direction, and the slide pin 136 is matched with the position of the slider guide grove 240B. When the slider 132 is moved forwards to move the slider pin 136 to the standard protruding position P5 at the forward end of the slider guide groove 240B, the manipulating wire 20 and the clips 12 and the like connected thereto move to the forward end side by the length of the linear portion of the slide guide groove 240B.

As described above, the length of the linear portion of the slider guide groove 240B is matched with the movement amount of the manipulating wire 20 until the foremost clip 12A protrudes from the forward end of the sheath 16 and the skirt portion 38 of the connection ring 14 is opened. Thus, by moving the slider 132 until the slider pin 136 reaches the standard protruding position P5, the foremost clip 12A can be placed in a usable state (standby state), as illustrated in FIG. 17D.

In the case where the skirt portion 38 of the connection ring 14A corresponding to the foremost clip 12A is not opened and the clip 12A is not placed in a usable state even when the slider pin 136 is moved to the standard protruding position P5 at the forward end of the slider guide grove 240B, the manipulating wire 20 and the clips 12 and the like connected thereto are moved to the forward end side by the length in the extension direction of the slider guide 238 of the curved portion 241 of the slider guide grove 240B by rotating the slider 132 clockwise viewed from the proximal end side to move the slider pin 136 from the standard protruding position P5 at the forward end of the slider guide groove 240B to the fine-adjustment protruding position P5'. Thus, the foremost clip 12A is allowed to protrude completely from the sheath 16 and placed in a usable state.

When the slider 132 is moved rearwards, in the manipulation operating portion of the forward end of the sheath 16, the clamping of the foremost clip 12A with the connection ring 14A as illustrated in FIG. 17E and the detachment from the second clip 12B as illustrated in FIG. 17F are performed at a time when the slider pin 136 moves to the clipping complete position P6. Thus, the clipping of the first clip 12A is completed.

Then, the slider 132 is moved rearwards, and the slider pin 136 is returned to the same position P7 as the home position P4. For the next clipping, the slider guide 238 is rotated by 90° in the same direction as mentioned above, whereby the slider pin 136 is matched with the home position P8 of the slider guide groove 240C.

As described above, the linear portion 239 of the slider guide groove 240C and the linear portion 239 of the slider guide groove 240D are respectively matched with the movement amounts of the manipulating wire 20 for allowing the second and third clips 12B, 12C to protrude from the sheath 16. Hereinafter, in the same way as in the first clipping, the slider 132 is moved forwards and rearwards, and the slider guide 238 is rotated by 90° in this order, whereby the second and third clipping manipulations can be performed.

In the case where the skirt portion 38 of the connection ring 14B corresponding to the clip 12B is not opened and the clip 12B is not placed in a usable state even when the slider pin 136 is moved to the standard protruding position P9 at the forward end of the slider guide groove 240C in the second clip 12B, the slider 132 may be rotated clockwise from the proximal end side to move the slider pin 136 from the standard protruding position P9 of the slider guide groove 240C to the fine-adjustment protruding position P9', and the manipulating wire 20 and the clips 12 and the like connected thereto may be moved to the forward end side by the length in the extension direction of the slider guide 239 of the curved portion 241 of the slider guide groove 240C, whereby the clip 12B is allowed to protrude completely from the sheath 16 and placed in a usable state.

The same applies to the third clip 12C.

The slider pin 136 may be composed so that it automatically returns from the fine-adjustment protruding position to the standard protruding position of the slider guide groove 240 with a restoring force of the slider guide 238.

### Embodiment 5

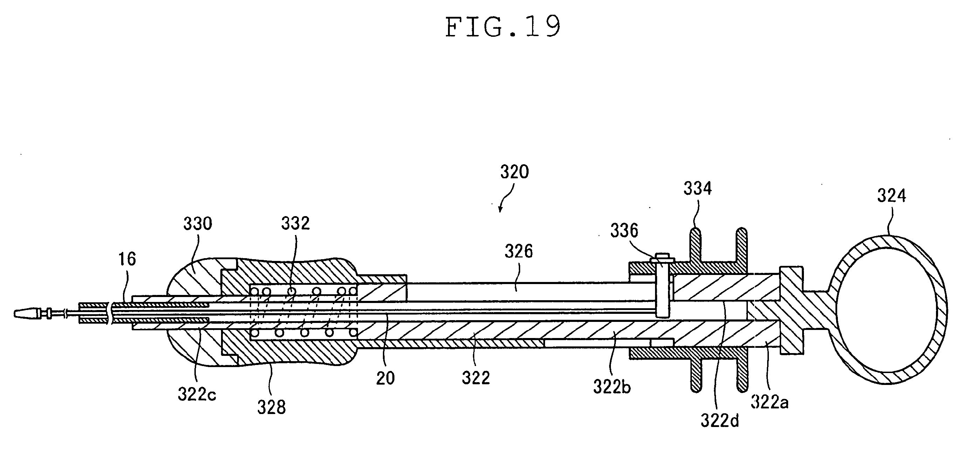

In Embodiment 5, a manipulating portion 320 as illustrated in FIG. 19 is used instead of the manipulating portion 120 according to Embodiment 2.

The manipulating portion 320 has a handle main body 322. The handle main body 322 is a stepped cylindrical member having three cylinder portions differing in outer diameter, and is formed, from the proximal end side, by a large diameter portion 322a, a medium diameter portion 322b, and a small diameter portion 322c.

The handle main body 322 has a through-hole 322d extending through the large diameter portion 322a, the medium diameter portion 322b, and the small diameter portion 322c. A finger hook ring 324 partially fitted in the through-hole 322d is fixed to the end portion of the large diameter portion 322a on the proximal end side.

The medium diameter portion 322b of the handle main body 322 has an engagement groove 326 which extends in the central axis direction and connects to the through-hole 322d.

A slider guide 328 in a substantially cylindrical shape is placed rotatably around the outer circumferential portion of the medium diameter portion 322b of the handle main body 322, and a position regulating member 330 is fixed to the outer circumferential portion of the small diameter portion 322c. Further, a coil spring 322 is placed between the stepped portion of the medium diameter portion 322b and the small diameter portion 322c of the handle main body 322, and the inner surface of the slider guide 328, and the slider guide 328 is pressurized against the position regulating member 330 due to the action of the coil spring 332.

The slider guide 328 has the same configuration as that of the slider guide 138 used in Embodiment 2 mentioned above. More specifically, the forward end portion of the slider guide 328 has four projections as illustrated in FIG. 13A, and the cylindrical portion on the proximal end side has five sides which are parallel to the plane orthogonal to the central axis and are different in position in the central axis direction as illustrated in FIG. 13B.

The position regulating member 330 has four projections as illustrated in FIG. 14 in the same way as in the position regulating member 142 used in Embodiment 2.

The four projections formed on the slider guide 328 and the four projections formed on the position regulating member 330 are engaged with each other, and pressurized with the coil spring 332, whereby the slider guide 328 can be prevented from rotating accidentally.

Further, five sides of the slider guide 328 are formed so that the engagement groove 326 of the handle main body 322 is overlapped with the position in the circumferential direction when the projections of the position regulating member 330 are engaged with the projections of the slider guide 328.

A substantially cylindrical slider 334 is movably placed in the axial direction on the outer circumferential portion of the handle main body 322 and the slider guide 328, and a slider pin 336 is fixed to the slider 334. The slider pin 336 reaches the inside of the through-hole 322d of the handle main body 322 through the engagement groove 326.

Further, the proximal end side portion of the sheath 16 is inserted to be fixed to the forward end of the small diameter portion 322c of the handle main body 322. The manipulating wire 20 is inserted into the sheath 16 and fixed to the slider pin 336 through the small diameter portion 322c and the medium diameter portion 322b of the handle main body 322.

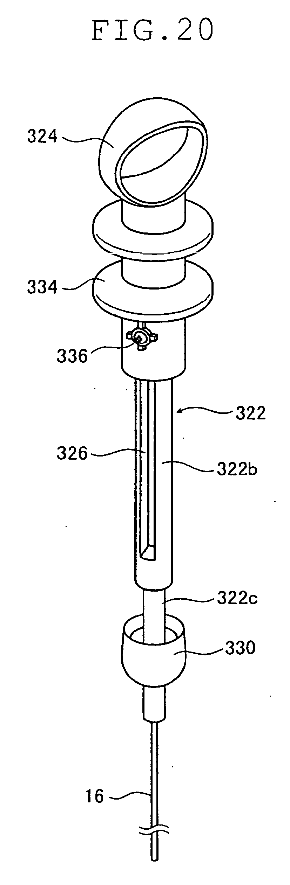

FIG. 20 illustrates a manipulating portion 320 with the slider guide 328 removed therefrom.

As in Embodiment 2 in which the movement amount of the slider 132 is regulated by the five sides of the slider guide 138 while the slider pin 136 is being guided between two bar members 122b of the main body rail 122, the movement amount of the slider 334 is regulated by the five sides of the slider guide 328 by moving the slider 334 while guiding the slider pin 336 by the engagement groove 326.

Even using the manipulating portion 320 with such a configuration, three clips are allowed to protrude successively from the sheath 16 only by the advancement and retreat manipulation of the manipulating wire 20 to perform each clipping in the same way as in Embodiment 2.

Means for matching the position of the engagement groove 326 of the handle main body 322 with that of the slider guide 328 in the circumferential direction is not limited to the configuration of engaging the projections of the position regulating member 330 with the projections of the slider guide 328 as in the illustrated example, and various known rotation position regulating means such as means for using a ratchet structure and means for using a so-called ball plunger and recesses can be used.

The successive clipping device of the present invention described in detail above should not be construed restrictively. It goes without saying that various improvements and variations are possible without departing from the scope of the claims. The successive clipping device of the present invention is applicable not only to a soft endoscope but also to a hard endoscope.

According to the above-mentioned embodiments of the present invention, there are disclosed the technical ideas as set forth in the following items:

### (Item 1)

The slide mechanism comprises a positioning pipe, fixed to the manipulating portion main body, in which a plurality of notches for pulling out the sheath by the first movement amount and the second movement amount are formed apart from each other in a pull-out direction of the sheath;
the sheath manipulating handle comprises a claw that is engaged selectively with any of the plurality of notches of the positioning pipe; and
a pull-out amount of the sheath is determined by selecting one of the plurality of notches with which the claw of the sheath manipulating handle is engaged.

### (Item 2)

Arc-shaped grooves connected to respective notches are formed in the positioning pipe; and
the claw to be engaged with the respective notches is guided along the grooves so that the sheath is rotated.

### (Item 3)

The manipulating portion comprises an adjustment means for moving a connection position of a proximal end of the manipulating wire with respect to the manipulating portion main body in an extension direction of the manipulating wire so as to finely adjust a relative position between the sheath and the manipulating wire.

### (Item 4)

The plurality of guide grooves in the proximal end portion of the movement amount regulating member respectively have a linear portion extending in an axial direction and a curved portion connected to a bottom portion of the linear portion and curved in the circumferential direction and the axial direction; and
the slider is moved in the extension direction of the sheath until the slider pin reaches the bottom portion of the linear portion, and thereafter, the slider is moved further in the circumferential direction and in the extension direction of the sheath so as to allow the slider pin to move along the curved portion so that a relative position between the sheath and the manipulating wire is finely adjusted.

## Claims

1. A successive clipping device, comprising:
a sheat (16);
a plurality of clip (12) loaded onto a forward end portion of the sheath while being engaged with another clips connected respectively in front and back directions;
a plurality of connection rings (14) corresponding to the plurality of clips and fitted into the sheath so as to be capable of advancing and retreating, and each covering an engagement portion between corresponding clips to maintain the plurality of clips in a connected state;
a manipulating wire (20) connected to a rearmost clip and pulling out a clip string formed of the plurality of clips; and
a manipulating portion (120) connected to a proximal end side of the sheath, which, regarding a foremost clip sinking in the sheath, moves the sheath and the manipulating wire relatively by a first movement amount required for allowing the foremost clip to protrude from a forward end of the sheath so as to place the foremost clip in a usable state, and which, regarding a subsequent clip, extrudes the manipulating wire with respect to the sheath by a second movement amount that is smaller than the first movement amount and required for allowing the subsequent clip to protrude from the forward end of the sheath so as to place the subsequent clip in a usable state,
**characterized in that**
the manipulating portion comprises:
a manipulating portion main body fixed to a proximal end portion of the sheath (122);
a slider (132) which a proximal end of the manipulating wire is connected and which is placed movably in an extension direction of the sheath with respect to the manipulating portion main body;
a substantially cylindrical movement amount regulating member (138) which is placed rotatably in a circumferential direction around an outer circumferential portion of the manipulating portion main body and has a proximal portion with a plurality of guide portions (92) to regulate a movement amount of the slider in the extension direction of the sheath in accordance with a rotation position in the circumferential direction; and
a slider pin (136) fixed to said slider and extending in a diameter direction of the movement amount regulating member;
wherein the slider is moved in the extension direction of the sheath until the slider pin reaches a bottom portion of any of the plurality of guide portions (92) formed in the proximal end portion of the movement amount regulating member to determine an extrusion amount of the manipulating wire.

2. The successive clipping device according to claim 1, wherein:
the proximal end portion of the movement amount regulating member has a plurality of stepped portions as the guide portion (92) along a periphery; and
the slide (132) is moved in the extension direction of the sheath until the slider pin (136) abuts any of the stepped portions of the proximal end portion of the movement amount regulating member to determine an extrusion amount of the manipulating wire.

3. The successive clipping device according to claim 2, wherein the stepped portions (92) in the proximal end portion of the movement amount regulating member include a first stepped portion with an axial direction length corresponding to the first movement amount and a second stepped portion with an axial direction length corresponding to the second movement amount.

4. The successive clipping device according to claim 1, wherein:
the proximal end portion of the movement amount regulating member has a plurality of guide grooves (240) having different lengths respectively in an axial direction as the guide portions; and
the slider is moved in the extension direction of the sheath until the slider pin reaches a bottom portion of any of the plurality of guide grooves having different lengths in the proximal end portion of the movement amount regulating member to determine an extrusion amount of the manipulating wire.

5. The successive clipping device according to claim 4, wherein the plurality of guide grooves (240) in the proximal end portion of the movement amount regulating member include at least three grooves whose difference in length corresponds to the first movement amount and the second movement amount.

6. The successive clipping device according to any one of claims 1 to 5, wherein the second movement amount is set to be a load interval of the clips in the sheath, and the first movement amount is set to be a length obtained by adding a distance between the forward end of the foremost clip having sunk in the sheath and the forward end of the sheath to the second movement amount.

7. A manipulating handle for manipulating a successive clipping device according to any one of claims 1 to 6, comprising:
a handle main body (122) fixed to the proximal end portion of the sheath;
the manipulating wire (20) having a forward end connected to the plurality of clips and the proximal end inserted in the sheath and the handle main body;
the slider (132) to which the proximal end of the manipulating wire is connected and which is placed movably in an extension direction of the sheath with respect to the handle main body; and
the substantially cylindrical movement amount regulating member (138) which is placed rotatably in a circumferential direction around an outer circumferential portion of the handle main body and which has the proximal portion with the plurality of guide portions (92) to regulate a movement amount of the slider in the extension direction of the sheath in accordance with a rotation position in the circumferential direction and
the slider pin fixing (136) in said slider and extending in a diameter direction of the movement amount regulating member.

8. The manipulating handle according to claim 7, wherein:
the proximal end portion of the movement amount regulating member (138) has a plurality of stepped portions (92) as the guide portions along a periphery; and
the slider (132) is moved in the extension direction of the sheath until the slider pin (136) abuts any of the stepped portions of the proximal end portion of the movement amount regulating member to determine an extrusion amount of the manipulating wire with respect to the sheath.

9. The manipulating handle according to claim 8, wherein the number of stepped portions in the proximal end portion of the movement amount regulating member is the same as the number of the plurality of clips connected to the forward end of the manipulating wire.

10. The manipulating handle according to claim 9, wherein the stepped portions in the proximal end portion of the movement amount regulating member include a first stepped portion with an axial direction length corresponding to a first movement amount of the manipulating wire with respect to the sheath required for allowing a foremost clip sinking in the sheath to protrude from a forward end of the sheath and placing the foremost clip in a usable state and a second stepped portion with an axial direction length corresponding to a second movement amount of the manipulating wire with respect to the sheath required for allowing a subsequent clip to protrude from the forward end of the sheath and placing the subsequent clip in a usable state.

11. The manipulating handle according to any one of claims 8 to 10, wherein the handle main body comprises an engagement groove (326) which extends in the extension direction of the sheath and guides a movement of the slider pin.

12. The manipulating handle according to claim 11, further comprising a position regulating means for regulating a rotation position in the circumferential direction of the movement amount regulating member so that a position in the circumferential direction of the engagement groove of the handle main body is matched with that of any of the stepped portions in the proximal end portion of the movement amount regulating member.

## Patentansprüche

1. Sukzessiv-Klammergerät, umfassend:
eine Hülse (16),
eine Mehrzahl von Klammern (12), die in einen vorderen Endbereich der Hülse eingebracht sind, wobei sie mit anderen Klammern in Vorwärts- und Rückwärtsrichtung verbunden sind;
eine Mehrzahl von Verbindungsringen (14) entsprechend der Mehrzahl von Klammern, eingepasst in die Hülse derart, dass sie vorgerückt und zurückgezogen werden können, wobei sie jeweils einen Eingriffsbereich zwischen entsprechenden Klammern bedecken, um die mehreren Klammern in einem verbundenen Zustand zu halten;
einen an eine hinterste Klammer gekoppelten Handhabungsdraht (20) zum Ausziehen einer durch die mehreren Klammern gebildeten Klammerkette; und
einen Handhabungsteil (120), der an eine proximale Endseite der Hülse gekoppelt ist, der in Bezug auf die vorderste, in der Hülse versenkten Klammer die Hülse und den Handhabungsdraht relativ um einen ersten Bewegungshub bewegt, welcher erforderlich ist, damit die vorderste Klammer von einem vorderen Ende der Hülse vorstehen kann, um die vorderste Klammer in einen Einsatzzustand zu platzieren, und der in Bezug auf eine nachfolgende Klammer den Handhabungsdraht in Bezug auf die Hülse um einen zweiten Bewegungshub ausfährt, der kleiner ist als der erste Bewegungshub und erforderlich ist, um der nachfolgenden Klammer zu ermöglichen, aus dem vorderen Ende der Hülse vorzustehen, um die nachfolgende Klammer in einen Einsatzzustand zu platzieren,
**dadurch gekennzeichnet, dass** der Handhabungsteil aufweist:
einen Handhabungsteil-Hauptkörper, der an einem proximalen Endteil der Hülse (122) fixiert ist,
einen Gleiter (132), an welchem ein proximales Ende des Handhabungsdrahts gekoppelt ist, und der beweglich in einer Erstreckungsrichtung der Hülse in Bezug auf den Handhabungsteil-Hauptkörper platziert ist;
ein im wesentlichen zylindrisches Bewegungshub-Regulierelement (138), welches drehbar in Umfangsrichtung um einen Aussenumfangsbereich des Handhabungsteil-Hauptkörpers angebracht ist und einen proximalen Abschnitt mit einer Mehrzahl von Führungsteilen (92) zum Regulieren eines Bewegungshubs des Gleiters in Erstreckungsrichtung der Hülse nach Maßgabe einer Drehstelllung in Umfangsrichtung besitzt; und
einen Gleiterstift (136), der an dem Gleiter fixiert ist und sich in einer Durchmesserrichtung des Bewegungshub-Regulierelements erstreckt;
wobei der Gleiter in der Erstreckungsrichtung der Hülse bewegt wird, bis der Gleiterstift einen Bodenteil eines der mehreren Führungsteile (92) erreicht, die in dem proximalen Endbereich des Bewegungshub-Regulierelements ausgebildet sind, um einen Ausfahrhub des Handhabungsdrahts festzulegen.

2. Klammergerät nach Anspruch 1, bei dem:
der proximale Endabschnitt des Bewegungshub-Regulierelements eine Mehrzahl abgestufter Abschnitte als die Führungsteile (92) entlang eines Umfangs besitzt; und
der Gleiter (132) in Erstreckungsrichtung der Hülse bewegt wird, bis der Gleiterstift (136) an einem der abgestuften Bereiche des proximalen Endabschnitts des Bewegungshub-Regulierelements anschlägt, um einen Ausfahrhub des Handhabungsdrahts zu bestimmen.

3. Klammergerät nach Anspruch 2, bei dem die abgestuften Bereiche (92) in dem proximalen Endabschnitt des Bewegungshub-Regulierelements einen ersten abgestuften Abschnitt mit einer Axialrichtungslänge enthalten, welche dem ersten Bewegungshub entspricht, und einen zweiten abgestuften Abschnitt mit einer Axialrichtungslänge aufweisen, die dem zweiten Bewegungshub entspricht.

4. Klammergerät nach Anspruch 1, bei dem:
der proximale Endabschnitt des Bewegungshub-Regulierelements eine Mehrzahl von Führungsnuten (240) unterschiedlicher Längen in axialer Richtung als die Führungsteile enthält; und
der Gleiter in der Erstreckungsrichtung der Hülse bewegt wird, bis der Gleiterstift einen Bodenbereich in einer der mehreren Führungsnuten unterschiedlicher Längen in dem proximalen Endabschnitt des Bewegungshub-Regulierelements erreicht, um einen Ausfahrhub des Handhabungsdrahts zu bestimmen.

5. Klammergerät nach Anspruch 4, bei dem die mehreren Führungsnuten (240) in dem proximalen Endabschnitt des Bewegungshub-Regulierelements mindestens drei Nuten beinhalten, deren Längenunterschied dem ersten Bewegungshub und dem zweiten Bewegungshub entspricht.

6. Klammergerät nach einem der Ansprüche 1 bis 5, bei dem der zweite Bewegungshub so eingestellt ist, dass er einen Ladeintervall der Klammern in der Hülse entspricht und der erste Bewegungshub so eingestellt wird, dass er eine Länge bildet, die er halten wird durch addieren eines Abstandes zwischen dem vorderen Ende der vordersten in die Hülse versenkten Klammer und dem vorderen Ende der Hülse, auf den zweiten Bewegungsschub.

7. Handhabe zum Handhaben eines Sukzessiv-Klammergeräts nach einem der Ansprüche 1 bis 6 umfassend:
einen Handhabe-Hauptkörper (122), der an dem proximalen Endabschnitt der Hülse fixiert ist;
den Handhabungsdraht (20), der mit einem vorderen Ende an den mehreren Klammern gekoppelt ist, und dessen proximales Ende in die Hülse und in den Handhabe-Hauptkörper eingesetzt ist;
den Gleiter (132), an welchen das proximale Ende des Handhabungsdrahts angebracht ist, und der beweglich in Erstreckungsrichtung der Hülse in Bezug auf den Handhabe-Hauptkörper platziert ist; und
das im Wesentlichen zylindrische Bewegungshub-Regulierelement (138), welches drehbar in Umfangsrichtung um einen Aussenumfangsbereich des Handhabe-Hauptkörpers platziert ist und welches den proximalen Teil mit den mehreren Führungsteilen (92) zum Regulieren eines Bewegungshubs des Gleiters in Erstreckungsrichtung der Hülse nach Maßgabe einer Drehstellung in Umfangsrichtung aufweist, und
den Gleiterstift (136), der den Gleiter fixiert und sich in einer Durchmesserrichtung des Bewegungshub-Regulierelements erstreckt.

8. Handhabe nach Anspruch 7, bei der
der proximale Endabschnitt des Bewegungshub-Regulierelements (138) eine Mehrzahl abgestufter Abschnitte (92) als Führungsteile entlang eines Umfangs aufweist; und
der Gleiter (132) in Erstreckungsrichtung der Hülse bewegt wird, bis der Gleiterstift (136) an einen der abgestuften Abschnitte des proximalen Endabschnitts des Bewegungshub-Regulierelements anschlägt, um einen Ausfahrhub des Handhabungsdrahts in Bezug auf die Hülse festzulegen.

9. Handhabe nach Anspruch 8, bei der die Anzahl abgestufter Abschnitte in dem proximalen Endabschnitt des Bewegungshub-Regulierelements, die gleich ist wie die Anzahl der mehreren Klammern, die an dem vorderen Ende des Handhabungsdrahts angebracht sind.

10. Handhabe nach Anspruch 9, bei der die abgestuften Abschnitte in dem proximalen Endabschnitt des Bewegungshub-Regulierelements einen ersten abgestuften Abschnitt mit einer Axialrichtungslänge entsprechend einen ersten Bewegungshub des Handhabungsdrahts in Bezug auf die Hülse enthalten, welche erforderlich ist, damit eine erste in die Hülse versenkte Klammer von einem vorderen Ende der Hülse vorstehen kann und die vorderste Klammer in einen Einsatzzustand platziert werden kann, und einen zweiten abgestuften Abschnitt mit einer Axialrichtungslänge entsprechend einem zweiten Bewegungshub des Handhabungsdrahts in Bezug auf die Hülse enthalten, der erforderlich ist, damit eine nachfolgende Klammer von dem vorderen Ende der Hülse vorstehen kann und die nachfolgende Klammer in einen Einsatzzustand platziert werden kann.

11. Handhabe nach einem der Ansprüche 8 bis 10, bei der der Handhabe-Hauptkörper eine Eingriffsnut (326) aufweist, die sich in Erstreckungsrichtung der Hülse erstreckt und eine Bewegung des Gleiterstifts führt.

12. Handhabe nach Anspruch 11, weiterhin umfassend eine Positionsreguliereinrichtung zum Regulieren einer Drehstellung in Umfangsrichtung des Bewegungshub-Regulierelements, so dass eine Stellung in Umfangsrichtung der Eingriffsnut des Handhabe-Hauptkörpers in Übereinstimmung gebracht wird mit der Lage eines der abgestuften Abschnitte in dem proximalen Endabschnitt des Bewegungshub-Regulierelements.

## Revendications

1. Dispositif pour l'application successive de clips médicaux, comprenant :
une gaine (16) ;
une pluralité de clips (12) chargés sur une portion à l'extrémité avant de la gaine tout en étant engagés avec d'autres clips connectés respectivement dans des directions vers l'avant et vers l'arrière ;
une pluralité de bagues de connexion (14) correspondant à la pluralité de clips et engagées dans la gaine de manière à être capable d'avancer et de reculer, couvrant chacune une portion d'engagement entre des clips correspondants pour maintenir la pluralité de clips dans un état connecté ;
un fil de manipulation (20) connecté à un clip tout à fait à l'arrière et tirant un ruban de clips formé d'une pluralité de clips ; et
une portion de manipulation (120) connectée sur un côté à l'extrémité proximale de la gaine qui, concernant un clip tout à fait à l'avant qui plonge dans la gaine, déplace la gaine et le fil de manipulation relativement sur une première distance de mouvement requise pour permettre au clip tout à fait à l'avant de se projeter depuis une extrémité antérieure de la gaine de façon à placer le clip tout à fait à l'avant dans un état capable d'être utilisé et qui, pour ce qui concerne un clip suivant, extrude le fil de manipulation par rapport à la gaine sur une seconde distance de mouvement qui est inférieure à la première distance de mouvement et qui est requise pour permettre au clip suivant de se projeter de l'extrémité antérieure de la gaine de façon à placer le clip suivant dans un état capable d'être utilisé,
**caractérisé en ce que**
la portion de manipulation comprend :
un corps principal de portion de manipulation fixé sur une portion à l'extrémité proximale de la gaine (122),
un coulisseau (132), auquel est connectée une extrémité proximale du fil de manipulation et qui est placé de façon mobile dans une direction d'extension de la gaine par rapport au corps principal de la portion de manipulation ;
un élément de régulation sensiblement cylindrique (138) pour la distance de mouvement, qui est placé en rotation dans une direction circonférentielle autour d'une portion circonférentielle extérieure du corps principal de la portion de manipulation et qui possède une portion proximale avec une pluralité de portions de guidage (92) pour réguler une distance de mouvement du coulisseau dans la direction d'extension de la gaine en accord avec une position en rotation dans la direction circonférentielle ; et
une tige de coulisseau (136) fixée sur ledit coulisseau et s'étendant dans une direction en diamètre de l'élément de régulation de distance de mouvement ;
dans lequel le coulisseau est déplacé dans la direction d'extension de la gaine jusqu'à ce que la tige de coulisseau atteigne une portion inférieure de l'une quelconque de la pluralité de portions de guidage (92) formées dans la portion d'extrémité proximale de l'élément de régulation de distance de mouvement pour déterminer une distance d'extrusion du fil de manipulation.

2. Dispositif pour l'application successive de clips médicaux selon la revendication 1, dans lequel :
la portion d'extrémité proximale de l'élément de régulation de distance de mouvement possède une pluralité de portions en gradins à titre de portions de guidage (92) le long d'une périphérie ; et
le coulisseau (132) est déplacé dans la direction d'extension de la gaine jusqu'à ce que la tige de coulisseau (136) vienne buter contre l'une quelconque des portions en gradins de la portion d'extrémité proximale de l'élément de régulation de distance de mouvement pour déterminer une distance d'extrusion du fil de manipulation.

3. Dispositif pour l'application successive de clips médicaux selon la revendication 2, dans lequel les portions en gradins (92) dans la portion d'extrémité proximale de l'élément de régulation de distance de mouvement incluent une première portion en gradin avec une longueur en direction axiale correspondant à la première distance de mouvement et une seconde portion en gradin avec une longueur en direction axiale qui correspond à la seconde distance de mouvement.

4. Dispositif pour l'application successive de clips médicaux selon la revendication 1, dans lequel :
la portion d'extrémité proximale de l'élément de régulation de distance de mouvement possède une pluralité de gorges de guidage (240) avec des longueurs différentes respectivement dans une direction axiale à titre de portions de guidage ; et
le coulisseau est déplacé dans la direction d'extension de la gaine jusqu'à ce que la tige de coulisseau atteigne une portion au fond de l'une quelconque de la pluralité de gorges de guidage ayant des longueurs différentes dans la portion d'extrémité proximale de l'élément de régulation de distance de mouvement pour déterminer une distance d'extrusion du fil de manipulation.

5. Dispositif pour l'application successive de clips médicaux selon la revendication 4, dans lequel la pluralité de gorges de guidage (240) dans la portion d'extrémité proximale de l'élément de régulation de distance de mouvement incluent au moins trois gorges dont la différence de longueur correspond à la première distance de mouvement et à la seconde distance de mouvement.

6. Dispositif pour l'application successive de clips médicaux selon l'une quelconque des revendications 1 à 5, dans lequel la seconde distance de mouvement est fixée pour être égale à un intervalle de chargement des clips dans la gaine, et la première distance de mouvement est fixée pour être égale à une longueur obtenue en ajoutant une distance entre l'extrémité antérieure du clip le plus à l'avant qui a plongé dans la gaine et l'extrémité antérieure de la gaine à la seconde distance de mouvement.

7. Poignée de manipulation pour manipuler un dispositif d'application successive de clips médicaux selon l'une quelconque des revendications 1 à 6, comprenant :
un corps principal de poignée (122) fixé sur la portion d'extrémité proximale de la gaine ;
le fil de manipulation (20) ayant une extrémité antérieure connectée à la pluralité de clips et l'extrémité proximale étant insérée dans la gaine et dans le corps principal de poignée ;
le coulisseau (132) auquel est connectée l'extrémité proximale du fil de manipulation et qui est placé de façon mobile dans une direction d'extension de la gaine par rapport au corps principal de poignée ; et
l'élément de régulation sensiblement cylindrique (138) pour la distance de mouvement, qui est placé en rotation dans une direction circonférentielle autour d'une portion circonférentielle extérieure du corps principal de poignée et qui possède la portion proximale avec la pluralité de portions de guidage (92) pour réguler une distance de mouvement du coulisseau dans la direction d'extension de la gaine en accord avec une position en rotation dans la direction circonférentielle ; et
la tige de coulisseau (136) fixée dans ledit coulisseau et s'étendant dans une direction en diamètre de l'élément de régulation de distance de mouvement.

8. Poignée de manipulation selon la revendication 7, dans laquelle :
la portion d'extrémité proximale de l'élément de régulation de distance de mouvement (138) possède une pluralité de portions en gradins (92) à titre de portions de guidage le long d'une périphérie ; et
le coulisseau (132) est déplacé dans la direction d'extension de la gaine jusqu'à ce que la tige de coulisseau (136) vienne buter contre l'une quelconque des portions en gradins de la portion d'extrémité proximale de l'élément de régulation de distance de mouvement pour déterminer une distance d'extrusion du fil de manipulation par rapport à la gaine.

9. Poignée de manipulation selon la revendication 8, dans laquelle le nombre de portions en gradins dans la portion d'extrémité proximale de l'élément de régulation de distance de mouvement est le même que le nombre de la pluralité de clips connectés à l'extrémité antérieure du fil de manipulation.

10. Poignée de manipulation selon la revendication 9, dans laquelle les portions en gradins dans la portion d'extrémité proximale de l'élément de régulation de distance de mouvement incluent une première portion en gradin avec une longueur en direction axiale correspondant à une première distance de mouvement du fil de manipulation par rapport à la gaine requise pour permettre à un clip le plus à l'avant qui plonge dans la gaine de se projeter depuis une extrémité antérieure de la gaine et placer le clip le plus à l'avant dans un état capable d'être utilisé, et une seconde portion en gradin avec une longueur en direction axiale correspondant à une seconde distance de mouvement du fil de manipulation par rapport à la gaine requise pour permettre à un clip suivant de se projeter de l'extrémité antérieure de la gaine et placer le clip suivant dans un état capable d'être utilisé.

11. Poignée de manipulation selon l'une quelconque des revendications 8 à 10, dans laquelle le corps principal de poignée comprend une gorge d'engagement (326) qui s'étend dans la direction d'extension de la gaine et qui guide un mouvement de la tige de coulisseau.

12. Poignée de manipulation selon la revendication 11, comprenant en outre des moyens de régulation de position pour réguler une position en rotation dans la direction circonférentielle de l'élément de régulation de distance de mouvement, de telle sorte qu'une position de la direction circonférentielle de la gorge d'engagement du corps principal de poignée est accordée avec celle de l'une quelconque des portions en gradins dans la portion d'extrémité proximale de l'élément de régulation de distance de mouvement.
